(19) Europäisches Patentamt
European Patent Office
Office européen des brevets

(11) EP 2 258 858 A1

(12) EUROPEAN PATENT APPLICATION

(43) Date of publication:
**08.12.2010 Bulletin 2010/49**

(51) Int Cl.:
***C12N 15/85*** *(2006.01)* ***A01K 67/027*** *(2006.01)*

(21) Application number: **09161995.7**

(22) Date of filing: **05.06.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(71) Applicant: **Universitätsklinikum Freiburg**
**79106 Freiburg (DE)**

(72) Inventors:
- **Schüle, Roland**
  **79367 Weisweil (DE)**
- **Günther, Thomas**
  **79104 Freiburg (DE)**
- **Metzger, Eric**
  **68600 Neuf-Brisach (FR)**

(74) Representative: **Kalhammer, Georg et al**
**Lederer & Keller**
**Patentanwälte**
**Prinzregentenstrasse 16**
**80538 München (DE)**

# (54) Transgenic LSD1 animal model for cancer

(57) The present invention relates to a non-human transgenic animal whose genome comprises a stably integrated transgenic nucleotide sequence encoding Lysine-specific Demethylase 1 (LSD1) operably linked to a promoter. The invention further concerns methods for generating the non-human animal and its use as a cancer model.



Printed by Jouve, 75001 PARIS (FR)

## Description

FIELD OF THE INVENTION

The present invention relates to an animal model for cancer which can be used to identify substances useful in the treatment of tumors and other proliverative disorders. In particular, the present invention relates to a non-human transgenic animal whose genome comprises a stably integrated transgenic nucleotide sequence encoding Lysine-specific Demethylase 1 (LSD1) operably linked to a promoter.

BACKGROUND OF THE INVENTION

### LSD1

The DNA of cells of higher organisms is complexed by histone proteins and organized in chromatin. For this reason the organization and the dynamic regulation of the chromatin structure plays an important role in the control of transcription. The agglomeration and removal, respectively, of modifications of the termini of core histones results in a selective transcriptional response. We are just beginning to understand that these modifications of the chromatin do not only control the development and the function of an organism, but are also of great importance regarding the formation and the progression of tumours (Kouzarides, 2007).

Modifications may be effected e.g. by methylating lysine residues (K). The methylation of histones has been considered enzymatically irreversible for decades (Kouzarides, 2007). The discovery of the function of "amine oxidase (flavin containing) domain 2" (LSD1), which was the first histone demethylation, has proven this dogma wrong just a few years ago. LSD1 is able to demethlyate mono- and dimethylated lysine 4 and 9 in histone 3 (HK4, or H3K9 respectively), which is associated to a promoter, in a flavin-dependent mechanism (Shi et al. 2004, Metzger et al. 2005, Lee et al. 2005).

LSD1 is expressed in numerous tissues and is necessary for the normal growth of the mouse embryo. The deletion of this gene through homologous recombination leads to an early embryonic lethality of homozygous deficient mice. This makes it more difficult to analyze the function of LSD1 under normal and pathological conditions.

### Prostate carcinoma

About 40,000 prostate carcinomas are newly diagnosed every year in Germany. Thus, this is the most frequent and most malignant carcinoma among men. The male sexual hormone, testosterone, plays an important role in the formation and the proliferation of the prostate carcinoma. Testosterone, or rather its active metabolite dihydroxytestosterone, executes its function via the androgen receptor (AR), which functions as ligand-dependent transcriptional factor in the nucleus and controls the differentiation, or the proliferation respectively, of normal prostate cells as well as of prostate carcinoma cells by controlling the expression of target genes. Therefore, the therapy of prostate carcinoma especially aims at decreasing the production of the male sexual hormone in the patients to inhibit the uncontrolled cell growth in the prostate gland. However, prostate carcinoma cells are also able to proliferate in the absence of androgens after some time (hormone refractory growth). At this stage of the disease, it is no longer possible to cure the patient (Sharifi et al., 2005).

### LSD1 controls the proliferation of prostate carcinoma cells

LSD associates with AR on the chromatized DNA in the prostate cells. Dependent on the androgen receptor, this complex leads to the removal of the androgen repressive H3K9 methyl-marker through LSD1. This results in the activation of target cells of the AR, which is linked with an increased proliferation of prostate cell carcinoma in the cell culture system (Metzger et al., 2005, Metzger et al., 2008).

In a normal prostate, LSD1 is expressed in the endothel. However, the amount of the LSD1 protein is significantly increased in prostate carcinoma and correlates directly proportional with the malignity of the tumour and thus can be used as a tumour marker. In this connection, the observation that the reduction of LSD1 protein or the inhibition of its enzymatic activity in the cell culture system leads to a reduced proliferation, is particularly significant. This effect is restricted to prostate carcinoma cell lines expressing AR and does not occur in AR-deficient cells, whereby the deceleration of the cell growth through targeted inhibition of LSD1 is not dependent from androgens, because AR cannot be activated through steroid hormones anymore due to a mutation in C4-2B cells (Metzger et al., 2005; Kahl et al., 2006; Metzger et al., 2008). Thus, LSD1 offers for the first time a possible basis for decelerating the growth of prostate carcinoma cells even in the hormone refractory phase. Chemicals which inhibit the activity of these proteins in the cell culture system have already been tested *in vitro* and in the cell culture system on their effectiveness.

The inventors' observation that also in e.g. lung carcinoma and in the cell culture lines which have been derived thereof an significantly higher amount of LSD1 protein is present relating to inconspicuous tissue, indicates a potential significance of LSD1 extending beyond the prostate carcinoma. Moreover, this fact indicates that LSD1 does not exclusively conduct the control over the gene expression through AR. This hypothesis is emphasized by the observation that LSD1 interacts e.g. with "transformation related protein 53" (p.53; Huang et al., 2007) and "retinoblastoma 1" (RB; Chau et al., 2008). Both gene products are essentially involved in controlling the cell cycle.

BRIEF DESCRIPTION OF THE DRAWINGS

Figure 1 depicts the demethylation of H3K4 catalyzed by LSD1. In a first step a histone (H3) is bound as a substrate and the methylated side chain is oxidized by the prostethic FAD group. Oxygen ($O_2$) is thereby reduced to $H_2O_2$. The resulting imine intermediate product is hydrolyzed, yielding a demethylated histone and formaldehyde (according to Forneris et al., 2008).

Figure 2 depicts the detection of human FLAG-tagged LSD1 protein in various tissues from Rosa26-LSD1 transgenic mice. Immunoprecipitated protein extracts from various tissues were separated according to their size and transferred to a membrane. The Western blot was carried out using an antibody directed against the FLAG tag. In the first lane transiently transfected FLAG-LSD1 protein has been run as a control.

Figure 3 depicts various tumors developed in adult Rosa26-LSD1 transgenic mice. Macroscopic (upper left panel) and microscopic (upper right panel) photographs of a bronchial adenocarcinoma are shown (arrows). Macroscopic image of a section through a liver lobe (bottom left panel) with hepatocellular carcinoma (arrow) is shown. The bottom right panel depicts a section through a peritoneal lipoma stained by haematoxylin and eosin.

Figure 4 depicts a vector map of a recombinant construct (targeting vector) for generating transgenic mice by microinjection, see examples *infra.*

SUMMARY OF THE INVENTION

The inventors of the subject application found that LSD1 alone is sufficient for the generation of carcinoma. This was shown by generating transgenic mice carrying flag-tagged human LSD1 under the control of the Rosa26 promoter (Zambrowicz et al., 1997). These transgenic mice ubiquitously overexpress LSD1 protein (see figure 2).

Therefore, the transgenic mice of the present invention are a valuable tool for studying established and potential anti-cancer agents.

The present invention therefore relates to a non-human transgenic animal whose genome comprises a stably integrated transgenic nucleotide sequence encoding Lysine-specific Demethylase 1 (LSD1) operably linked to a promoter. The present invention is directed to a transgenic non-human animal, preferably a transgenic non-human mammal, whose genome comprises a DNA sequence encoding human LSD1 (=hLSD1) or an active fragment or variant thereof, which is operably linked to an expression control sequence, wherein the expression of the hLSD1 in the mammal is effective in stimulating the generation and/or growth of tumor cells or tissue. The transgenic mammal is a preferably a rodent, more preferably a mouse. The hLSD1 is preferably wild type hLSD1, and the DNA sequence may encode an active fragment or variant of hLSD1.

In the above transgenic mammal, the expression control sequence preferably comprises a constitutive promoter which may or may not be tissue specific; an inducible/repressible promoter or control element is also included. A preferred expression control sequence comprises a mouse Rosa26 promoter.

The transgenic mammal may be hemizygous for hLSD1, or more preferably, double hemizygous for hLSD1. It is preferably fertile.

In one embodiment of the above transgenic mammal, the polynucleotide was introduced into the animal, or an ancestor thereof, at an embryonic stage.

Also provided is a cell, isolated from the transgenic mammal, or a progeny cell of the isolated cell.

Also included is a method for testing an agent for its ability to inhibit the growth or metastasis of a human tumor, comprising exposing a transgenic mammal of the invention to the test agent, before or after development of a tumor in the mammal, and determining the effect of the test agent on tumor development and/or growth.

Also provided is a method for evaluating the effect of a test agent or treatment as a potential therapy for cancer, comprising administering a test agent or treatment to a transgenic mammal as above, and comparing the growth or metastasis of the tumor cell or tissue to a control transgenic animal.

Another embodiment provides a method for producing a transgenic mouse which is transgenic for LSD1, comprising incorporating into the genome of a mouse, at least one site, a polynucleotide encoding LSD1, or a biologically active fragment or variant thereof, which is operably linked to an expression control sequence, wherein the expression of the LSD1 in the mouse is effective to support the development and/or growth of tumor or cancer cells or tissue.
Also included is a method for preparing hLSD1 produced in a transgenic non-human mammal, comprising collecting an hLSD1-containing biological sample from the transgenic mammal as above, preferably a mouse, wherein the sample may be serum or plasma, and the hLSD1 in the sample is optionally further enriched or purified.
The invention further relates to the use of a non-human transgenic animal described herein as an animal model for cancer, e.g. for lung cancer, hepatocellular carcinoma and/or peritoneal lipoma.
The invention further relates to a recombinant nucleic acid construct for generating a nonhuman transgenic animal, said construct comprising a nucleotide sequence encoding LSD1 operably linked to a promoter, e.g the Rosa26 promoter. Said nucleotide sequence may be flanked by nucleotide sequences homologous to target sequences in the target animal. A preferred recombinant nucleic acid construct has the sequence as shown in SEQ ID NO:6.

## DETAILED DESCRIPTION OF THE INVENTION

The present invention relates to an animal model for evaluating growth, survival and/or metastasis of tumor cells or tissue. For example, the invention provides a transgenic non-human vertebrate animal, preferably a mammal, preferably a rodent, such as a mouse. In a most preferred embodiment, the genome of the animal comprises a polynucleotide which expresses the human growth factor, hLSD1.
One aspect of the invention non-human transgenic mammal (e.g., a rodent, preferably a mouse) whose genome comprises a DNA sequence encoding hLSD1, or encoding a biologically active fragment or variant thereof, which is operably linked to an expression control sequence, wherein overexpression of the hLSD1 leads to the development of tumors in the transgenic animal.
Suitable animals are available, or easily generated, using conventional methods, in a variety of genera, including rodents (e.g., rats), rabbits, guinea pigs, dogs, goats, sheep, cows, horses, pigs, llamas, camels or the like. Preferably, the non-human transgenic animal is a transgenic mouse.
The animal from which the progeny animal is descended is referred to as "progenitor animal." "Progeny" of a progenitor mammal are any animals which are descended from the progenitor as a result of sexual reproduction or cloning of the progenitor, and which have inherited genetic material from the progenitor. In this context, cloning refers to production of genetically identical offspring from DNA or a cell(s) of the progenitor animal. As used herein, "development of an animal" from a cell or cells (embryonic cells, for example), or development of a cell or cells into an animal, refers to the developmental process that includes growth, division and differentiation of a fertilized egg or embryonic cells (and their progeny) to form an embryo, and birth and development of that embryonic animal into an adult animal.
An animal is "derived from" a transgenic ovum, sperm cell, embryo or other cell if the transgenic ovum, sperm cell, embryo or other cell contributes DNA to the animal's genomic DNA. For example, a transgenic embryo of the invention can develop into a transgenic animal of the invention. A transgenic ovum of the invention can be fertilized to create a transgenic embryo of the invention that develops into a transgenic animal of the invention. A transgenic sperm of the invention can be used to fertilize an ovum to create a transgenic embryo of the invention that develops into a transgenic animal of the invention. A transgenic cell of the invention can be used to clone a transgenic animal of the invention.
As used herein, a "transgenic non-human mammal" is a non-human mammal into which an exogenous recombinant construct has been introduced, or its progeny. Such a mammal may have developed from (a) embryonic cells into which the construct has been directly introduced or (b) progeny cells of (a). As used herein, an "exogenous construct" is a nucleic acid that is artificially introduced, or was originally artificially introduced, into an animal. The term "artificial introduction" excludes introduction of a construct into an animal through normal reproductive processes (such as by cross breeding). However, animals that have been produced by transfer of an exogenous construct through the breeding of a mammal comprising the construct (into whom the construct was originally "artificially introduced") are considered to "comprise the exogenous construct." Such animals are progeny of animals into which the exogenous construct has been introduced.
A non-human transgenic mammal of the invention is preferably one whose somatic and germ cells comprise at least one genomically integrated copy of a recombinant construct of the invention (a recombinant construct comprising a sequence encoding LSD, preferably hLSD1), or an active fragment or variant thereof, which sequence is operably linked to an expression control sequence. Alternatively, the disclosed transgene construct can also be assembled as an artificial chromosome, which does not integrate into the genome but which is maintained and inherited substantially stably in the animal. Artificial chromosomes of more than 200 kb can be used for this purpose.
The invention further provides a transgenic gamete, including a transgenic ovum or sperm cell, a transgenic embryo, and any other type of transgenic cell or cluster of cells, whether haploid, diploid, or of higher zygosity having at least one genomically integrated copy of a recombinant construct of the invention. The transgenic gamete, ovum, sperm cell, embryo, somatic cell or animal cell, may comprise two or more copies of the transgene. These are preferably tandemly arranged or may be inserted at noncontiguous sites in the haplotype (and genome).

As used herein, the term "embryo" includes a fertilized ovum or egg (i.e., a zygote) as well as later multicellular developmental stages of the organism. The recombinant construct is preferably integrated into the animal's somatic and germ cells, or is present in stable extrachromosomal form, such as an artificial chromosome, that is stable and heritable. The transgenic animal or cell preferably contains a multiplicity of genomically integrated copies of the construct. Preferably, multiple copies of the construct are integrated into the host's genome in a contiguous, head-to-tail orientation.

Also included herein are progeny of the transgenic animal that preferably comprise at least one genomically integrated copy of the construct, and transgenic animals derived from a transgenic ovum, sperm, embryo or other cell of the invention.

In some embodiments of the invention, the transgenic animal is sterile although, preferably, it is fertile. The present invention further includes a cell line derived from a transgenic embryo or other transgenic cell of the invention, which contains at least one copy of a recombinant construct of the invention. Methods of isolating such cells and propagating them are conventional.

While the mouse is preferred, the present invention includes other genera and species, such as other rodents (e.g., rats), rabbits, guinea pigs, dogs, goats, sheep, cows, pigs, llamas, camels, etc.

*Generation of transgenic animals*

The transgenic non-human animals of the invention are produced by introducing transgenes into the germline of the non-human animal. Embryonal target cells at various developmental stages are used to introduce the transgenes of the invention. Different methods are used depending on the stage of development of the embryonal target cell(s). Such methods include, but are not limited to, microinjection of zygotes, viral integration, and transformation of embryonic stem cells as described below.

Microinjection of zygotes is the preferred method for incorporating transgenes into animal genomes. A zygote, which is a fertilized ovum that has not undergone pronuclei fusion or subsequent cell division, is the preferred target cell for microinjection of transgenic DNA sequences. The murine male pronucleus reaches a size of approximately 20 micrometers in diameter, a feature which allows for the reproducible injection of 1-2 picoliters of a solution containing transgenic DNA sequences. The use of a zygote for introduction of transgenes has the advantage that, in most cases, the injected transgenic DNA sequences will be incorporated into the host animal's genome before the first cell division. Brinster et al., Proc. Natl. Acad. Sci. USA 82:4438 (1985). As a consequence, all cells of the resultant transgenic animals (founder animals) stably carry an incorporated transgene at a particular genetic locus.

Viral integration can also be used to introduce the transgenes of the invention into an animal. The developing embryos are cultured in vitro to the blastocyte developmental stage. The blastomeres may be infected with appropriate retroviruses. Jaenich, Proc. Natl. Acad. Sci. USA 73:1260. Infection of the blastomeres is enhanced by enzymatic removal of the zona pellucida. Transgenes are introduced via viral vectors which are typically replication-defective but which remain competent for integration of viral-associated DNA sequences, including transgenic DNA sequences linked to such viral sequences, into the host animal's genome. Transfection is easily and efficiently obtained by culture of blastomeres on a monolayer of cells producing the transgene-containing viral vector. Alternatively, infection may be performed using cells at a later developmental stage, such as blastocoeles. In any event, most transgenic founder animals produced by viral integration will be mosaics for the transgenic allele; that is, the transgene is incorporated into only a subset of all the cells that form the transgenic founder animals. Moreover, multiple viral integration events may occur in a single founder animal, generating multiple transgenic alleles which will segregate in future generations of offspring. Introduction of transgenes into germline cells by this method is possible but probably occurs at a low frequency. However, once a transgene has been introduced into germline cells by this method, offspring may be produced in which the transgenic allele is present in all of the animal's cells, i.e., in both somatic and germline cells.

Embryonic stem (ES) cells can also serve as target cells for introduction of the transgenes of the invention into animals. ES cells are obtained from pre-implantation embryos that are cultured *in vitro.* Evans et al., Nature 292:154 (1981). ES cells that have been transformed with a transgene can be combined with an animal blastocyst, after which the ES cells colonize the embryo and contribute to the germline of the resulting animal. Once a transgene has been introduced into germline cells by this method, offspring may be produced in which the transgenic allele is present in all of the animal's cells, i.e., in both somatic and germline cells.

The transgenic nucleic acid of the invention may be stably integrated into germ line cells and transmitted to offspring of the transgenic animal as Mendelian loci. Other transgenic techniques result in mosaic transgenic animals, in which some cells carry the transgenes and other cells do not. In mosaic transgenic animals in which germ line cells do not carry the transgenes, transmission of the transgenes to offspring does not occur. Nevertheless, mosaic transgenic animals are capable of demonstrating phenotypes associated with the transgenes.

In practicing the invention, animals of the transgenic maintenance line are crossed with animals having a genetic background in which expression of the transgene results in symptoms of tumor formation. Offspring that have inherited the transgenic nucleic acids of the invention are distinguished from littermates that have not inherited transgenic nucleic acids by analysis of genetic material from the offspring for the presence of nucleic acid sequences derived from the transgenic nucleic acids of the invention. For example, biological fluids that contain polypeptides uniquely encoded by the transgenic nucleic acids of the invention may be immunoassayed for the presence of the polypeptides. A simpler and more reliable means of identifying transgenic offspring comprises obtaining a tissue sample from an extremity of an animal, such as, for example, a tail, and analyzing the sample for the presence of nucleic acid sequences corresponding to the DNA sequence of a unique portion or portions of the transgenic nucleic acids of the invention. The presence of such nucleic acid sequences may be determined by, e.g., hybridization ("Southern") analysis with DNA sequences corresponding to unique portions of the transgene, analysis of the products of PCR reactions using DNA sequences in a sample as substrates, oligonucleotides derived from the transgene's DNA sequence, and the like.

The present invention is also directed to the creation of transgenic mice in whose tissue specific expression of the hLSD1 transgene is driven by a tissue specific promoter, as is discussed more extensively below.

*Targeting vectors*

As used herein, the term "polynucleotide" is interchangeable with "nucleic acid." A polynucleotide of the present invention may be recombinant, natural, or synthetic or semi-synthetic, or any combination thereof. Polynucleotides of the invention may be RNA, PNA, LNA, or DNA, or combinations thereof. As used herein, the terms peptide, polypeptide and protein are also interchangeable.

A "recombinant construct" (also referred to herein as a "construct" for short) or a "transgene" of "transgenic nucleic acid" which is used to generate a transgenic animal of the invention is a polynucleotide which comprises a sequence encoding LSD1 (preferably hLSD1), or an active fragment or variant thereof, which is operably linked to an expression control sequence. The coding sequence comprises LSD1 exon sequences, although it may optionally include intron sequences which are either derived from an hLSD1 genomic DNA or DNA of an unrelated chromosomal gene.

The recombinant construct may comprise a sequence encoding mLSD1 or at least a biologically active fragment thereof. Preferably, the recombinant construct comprises a sequence encoding human LSD1 (hLSD1) or a biologically active fragment thereof. The amino acid sequences of hLSD1 and mLSD1 are shown in SEQ ID NO:3 and 5, respectively. The nucleotide sequence encoding mLSD1 is shown in SEQ ID NO:4. The nucleotide sequence encoding hLSD1 is shown in SEQ ID NO:1. The hLSD1 cDNA sequence including 5'- and 3'- untranslated regions is shown in SEQ ID NO:2.

The nucleotide sequence of an exemplary recombinant construct is shown in SEQ ID NO:6. The amino acid sequence encoded by this construct is shown in SEQ ID NO:7.

A construct of the invention may comprise an "active fragment" or an "active variant" of a sequences encoding LSD1, e.g., hLSD1. Such an active fragment or variant encodes a form of LSD1 that exhibits at least a measurable degree of at least one biological activity of LSD1. For example, a polypeptide encoded by an "active" fragment or variant has enzymatic activity, in particular. A skilled worker can readily test whether a polynucleotide of interest exhibits this desired function, by employing well-known assays, such as those described elsewhere herein.

An active fragment of the invention may be of any size that is compatible with, for example, the requirement that it encode a polypeptide that can stimulate the growth of tumor cells. For example, an LSD1-encoding sequence can be shortened by about 20, about 40, or about 60 nucleotides, etc., provided that the encoded polypeptide retains the biological activity.

An active variant of the invention includes, for example, polynucleotides comprising a sequence that exhibit a sequence identity to DNA encoding wild type hLSD1, e.g., SEQ ID NO:1, of at least about 70%, preferably at least about 80%, more preferably at least about 90% or 95%, or 98%, provided that the polynucleotide encodes a polypeptide with the desired activity. In accordance with the present invention, a sequence being evaluated (the "Compared Sequence") has a certain "percent identity with," or is a certain "percent identical to" a claimed or described sequence (the "Reference Sequence") after alignment of the two sequences. The "Percent Identity" is determined according to the following formula:

$$\text{Percent Identity}=100[1-(C/R)]$$

In this formula, C is the number of differences between the Reference Sequence and the Compared Sequence over the length of alignment between the two sequences wherein (i) each base in the Reference Sequence that does not have a corresponding aligned base in the Compared Sequence, and (ii) each gap in the Reference Sequence, and (iii) each aligned base in the Reference Sequence that is different from an aligned base in the Compared Sequence constitutes a difference. R is the number of bases of the Reference Sequence over the length of the alignment with the Compared Sequence with any gap created in the Reference Sequence also being counted as a base.

If an alignment exists between the Compared Sequence and the Reference Sequence for which the Percent Identity (calculated as above) is about equal to, or greater than, a specified minimum, the Compared Sequence has that specified minimum Percent Identity even if alignments may exist elsewhere in the sequence that show a lower Percent Identity than that specified.

In a preferred embodiment, the length of aligned sequence for comparison purposes is at least 30%, preferably at least 40%, more preferably at least 50%, even more preferably at least 60%, and even more preferably at least 70%, 80%, or 90% of the length of the Reference Sequence.

The comparison of sequences and determination of percent identity (and percent similarity) between two amino acid sequences can be accomplished using any suitable program, e.g. the program "BLAST 2 SEQUENCES (blastp)" (Tatusova et al. (1999) FEMS Microbiol. Lett. 174, 247-250) with the following parameters: Matrix BLOSUM62; Open gap 11 and extension gap 1 penalties; gap x_dropoff50; expect 10.0 word size 3; Filter: none. According to the present invention, the sequence comparison covers at least 40 amino acids, preferably at least 80 amino acids, more preferably at least 100 amino acids, and most preferably at least 120 amino acids.

The degree of identity between two polynucleotide sequences can be determined by using the program "BLAST 2 SEQUENCES (blastn)" (Tatusova et al. (1999) FEMS Microbiol. Lett. 174, 247-250) with the following parameters: reward for a match 1; penalty for a mismatch -2; open gap 5 and extension gap 2 penalties; gap x_dropoff 50; expect 10.0; word size 11; filter; none. According to the present invention the sequence comparison covers at least 50 nucleotides, preferably at least 100 nucleotides, more preferably at least 200 nucleotides; most preferably at least 300 nucleotides.

An active variant of the invention may take any of a variety of forms, including, e.g., a naturally or non-naturally occurring polymorphisms, including single nucleotide polymorphisms (SNPs), allelic variants, and mutants. The variant may comprise one or more additions, insertions, deletions, substitutions, transitions, transversions, inversions, or chromosomal translocations or the like; the variant may result from an alternative splicing event. Any combination of the foregoing is also intended. Other types of active variants will be evident to a person skilled in the art. For example, the nucleotides of a polynucleotide can be joined by known linkages, e.g., ester, sulfamate, sulfamide, phosphorothioate, phosphoramidate, methylphosphonate, carbamate, etc., depending on the desired purpose, such as improved in vivo stability, etc. See, e.g., U.S. Pat. No. 5,378,825. Any desired nucleotide or nucleotide analog such as 6-mercaptoguanine, 8-oxoguanine, etc. can be incorporated.

Active variants or fragments of the invention also includes polynucleotides which encode LSD1 polypeptides that differ from wild type hLSD1, yet retain at least one of the hLSD1 functions noted above. For example, the polypeptide may comprise a sequence that differs from the wild type hLSD1 sequence by one or more conservative amino acid substitutions, or that is at least about 70% identical, preferably at least about 80%, 90%, 95% or 98% identical, to the wild type sequence. The wild type sequence of hLSD1 is encoded by the cDNA having the sequence SEQ ID NO:1.

In the present recombinant construct, a hLSD1 coding sequence, or active fragment or variant thereof, is operably linked to an "expression control sequence", which term means a polynucleotide sequence that regulates expression of a polypeptide from the coding sequence to which it is functionally ("operably") linked. Expression can be regulated at the level of transcription or translation. Thus, an expression control sequence may include transcriptional elements and translational elements. Such elements include promoters, domains within promoters, upstream elements, enhancers, elements that confer tissue- or cell-specificity, response elements, ribosome binding sequences, transcriptional terminators, etc. An expression control sequence is operably linked to a nucleotide coding sequence when it is positioned in such a manner to drive or control expression of the coding sequence. For example, a promoter operably linked 5' to a coding sequence drives expression of the coding sequence. One expression control sequence may be linked to another expression control sequence. For example, a tissue-specific expression control sequence may be linked to a basal promoter element.

Any of a variety of expression control sequences can be used in constructs of the invention. In preferred embodiments, the expression control sequence comprises a constitutive promoter, which is expressed in a wide variety of cell types. Many such suitable expression control sequences are well-known in the art. Among the suitable strong constitutive promoters and/or enhancers are expression control sequences from DNA viruses (e.g., SV40, polyoma virus, adenoviruses, adeno-associated virus, pox viruses, CMV, HSV, etc.) or from retroviral LTRs. Tissue-specific promoters well-known in the art maybe be used to direct expression of hLSD1 to specific cell lineages.

While the experiments discussed in the Examples below were conducted using the mouse Rosa26 gene promoter, other Rosa26-related promoters capable of directing LSD1 gene expression can be used to yield similar results as will be evident to those of skill in the art. An example is shorter Rosa26 5'-upstream sequences, which can nevertheless achieve the same degree of expression. Also useful are minor DNA sequence variants of the Rosa26 promoter, such as point mutations, partial deletions or chemical modifications.

The Rosa26 promoter is known to be expressible in rats, rabbits and humans, and may be expressed in any other mammalian species, a fact which may be determined by routine testing. In addition, sequences that are similar to the 5' flanking sequence of the mouse Rosa26 gene, including, but not limited to, promoters of Rosa26 homologues of other species (such as human, cattle, sheep, goat, rabbit and rat), can also be used. The Rosa26 gene is sufficiently conserved among different mammalian species that similar results with other Rosa26 promoters are expected.

For tissue-specific expression of the transgene in the transgenic animal, the coding sequence must be operably linked to an expression control sequence that drives expression specifically in that tissue. Suitable tissue-specific expression control sequences include the following: MMTV-LTR (for mammary-specific expression), etc.

*InducibleIRepressible Expression Control Systems*

An inducible promoter is one which, in response to the presence of an inducer, is activated. Hence, a coding sequence driven by an inducible promoter can be turned on or off by providing or withdrawing the inducer. A promoter may be homologous, derived from the same species as the coding sequence. Preferably, the promoter is heterologous, that is, derived from another species, or even from a virus. hLSD1 constructs in accordance with the present invention may be operably linked to an inducible or repressible control elements. An repressible system, described by Gossen, M. et al., Proc Natl Acad Sci USA 89:5547-51 (1992), is based on the use of control elements of the tetracycline-resistance operon encoded in Tn10 of E. coli. The tet repressor is fused with the activating domain of Herpes simplex virus VP16 to generate a tetracycline-controlled transactivator. Such a transactivator is used to stimulate transcription from a promoter sequence, such as the CMV promoter IE.

A gene controlled by a promoter acting under the influence of the tetracycline-controlled transactivator can be constitutively expressed and turned off by using an effective concentration of tetracycline. Such a system can regulate a gene over about five orders of magnitude. The tetracycline-repressible system functions in vivo in mice, where tetracycline administration via the diet is used to keep the expression of the inducible gene off. Tetracycline analogs which cross the blood-brain barrier can be used if gene activity is desired in the brain.

Two steps of transfection may be used to produce the appropriate system. A first transfection is used to isolate clones expressing the transactivator. The best clones are identified by testing each in a transient transfection assay for the ability to express a marker gene, such as an estrogen-dependent luciferase. The second transfection involves the hLSD1 coding sequence under control of an inducible promoter into a transactivator-containing clone. One strategy involves first isolating a stable cell line expressing the inducible hLSD1 protein or peptide by cotransfection of both plasmids into appropriate target cells. After selection, for example with G418, clones showing estrogen-dependent expression of hLSD1 may be detected by an immunoassay or biological assay. To increase the rate of plasmid integration and to stabilize the integrated plasmids in the host genome, the plasmids are preferably linearized and cotransfected into cells in the presence of mammalian high molecular weight DNA as a carrier.

The relative advantages of a two vector system, as described above, over a single vector system involving a larger plasmid is that in a two vector system, multiple copies of the reporter plasmid (encoding the gene of interest) may be needed to obtain a detectable biological effect in a cell, while one or only a few copies of the transactivator-carrying plasmid may suffice.

According to the present invention, the hLSD1 DNA molecule is placed under the control of a promoter subject to regulation by a tetracycline-controlled transactivator. Such a construct (in a single vector or preferably two vector form) is delivered into target cells, whether embryonic, adult normal or tumor, either in vitro or in vivo. To express the hLSD1, tetracycline is withheld so that the hLSD1 DNA is expressed. To prevent the action of the hLSD1, for example, locally, tetracycline or an active congener of tetracycline is administered locally to the cells transfected with the constructs. Effective systemic doses (oral or parenteral) of tetracycline are in the range of about 0.1 mg to 1 g per day. In a preferred embodiment, the transactivator is maintained in the "on" position by withholding tetracycline.

An estrogen-inducible system described by Braselmann, S. et al. Proc Natl Acad Sci USA (1993) 90:1657-61, is based on the fact that most mammalian cells neither express any Gal4-like activity nor endogenous estrogen receptor (ER), thus rendering estrogen an inert signal for them. The authors developed a selective induction system based on the estrogen-regulatable transcription factor Gal-ER. Gal-ER consists of the DNA-binding domain of the yeast Gal4 protein fused to the hormone-binding domain of the human ER and hence exclusively regulates a transfected coding sequence under the control of a Gal4-responsive promoter in mammalian cells. This system includes a synthetic Gal4-responsive promoter which consists of four Gal4-binding sites, an inverted CCAAT element, a TATA box, and the adenovirus major late initiation region. This promoter shows extremely low basal activity in the absence of, and high inducibility in the presence of, ligand-activated Gal-ER. The transcription factor Gal-ER is rendered more potent and less susceptible to cell type-specific variation by fusing the strong activating domain of the herpesvirus protein VP16 onto its C-terminus. In response to estrogen, e.g., 17-β estradiol, Gal-ER-VP16 may induce the Gal4-responsive promoter at least 100-fold in transfected cells. Thus, the Gal-ER induction system is a powerful genetic switch for regulating heterologous genes. For induction of expression of the DNA molecules of the present invention in an estrogen inducible system in an animal, local or systemic treatment with estrogen would be required. An effective dose of an estrogen is a dose which would trigger the expression of an hLSD1-encoding nucleic acid of the present invention to produce hLSD1 and promote growth of hLSD1-expressing tumor cells. Such doses can be ascertained by one skilled in the art. Preferably, doses in the range of about 0.05 to 100 mg/kg of an estrogen are used in a single dose or in multiple doses over a period of about one week days to about 6 months, or even longer. Forms and preparations of estrogen and their usage in animals, particularly in humans, are well-known in the art. Estrogen analogues which are capable of specifically activating the exogenous transactivator while having fewer biological effects and side effects are preferred.

Ionizing radiation has been used to activate the transcription of exogenous genes, for example, encoding a cytotoxic protein TNF-I (Weichselbaum, R R et al., Int J Radiation Oncology Biol Phys 24:565-67 (1992)) This may be accomplished through the use of radiation-responsive elements distal to the transcription start site of such genes. See, for example, Hallahan, D et al., Proc Natl Acad Sci USA 88:2152-20 (1991); Datta, R et al., Proc Natl Acad Sci USA 89: 10149-53 (1992); Weichselbaum et al., supra; Hallahan, D E et al. J Biol Chem 268:4903-07 (1993); Weichselbaum, R R et al., Intl J Radiation Oncology Bio. Phys 30:229-34 (1994); Hallahan, D E et al. Nature Med 1:786-91 (1995), which references are hereby incorporated by reference in their entirety. Thus, the present invention provides methods for the spatial and temporal control of gene expression with such radiation-inducible promoters to activate hLSD1. The hLSD1 coding sequence is placed in a vector under control of a radiation-inducible promoter.

Another generally applicable method is used in conjunction with gene therapy/gene delivery methods described below, for inducing activation of a gene of interest, in particular hLSD1. This method is disclosed in detail in PCT publications WO94/18317, WO95/02684 and WO95/05389; Spencer, D. M. et al., Science 262:1019-1024 (1993); Travis, Science 262:989 (1993); and Chem. & Eng. News, Nov. 15, 1993, pp. 55-57, which references are hereby incorporated by reference in their entirety. This approach uses intracellular protein homodimerization, heterodimerization and oligomerization in living cells into which the hLSD1 DNA has been transfected. Chimeric responder proteins are intracellularly expressed as fusion proteins with a specific receptor domain. Treatment of the cells with a cell-permeable multivalent ligand reagent which binds to the receptor domain leads to dimerization or oligomerization of the chimeric receptor. In analogy to other chimeric receptors (see e.g. Weiss, Cell (1993) 73, 209), the chimeric proteins are designed such that oligomerization triggers the desired subsequent events, e.g. the propagation of an intracellular signal via subsequent protein-protein interactions and thereby the activation of a specific subset of transcription factors. The initiation of transcription can be detected using a reporter gene assay. Intracellular crosslinking of chimeric proteins by synthetic ligands allows regulation of the synthesis of hLSD1 and, thereby, selective induction of tumor growth.

In a preferred embodiment, the expression control sequence (either a ubiquitously acting expression control sequence or a tissue-specific one) is expressed in a regulatable fashion, meaning that it is preferably a component of any of a number of well-known regulatable expression systems.

Methods of making recombinant constructs are conventional. Such methods, as well as many other molecular biological methods used in conjunction with the present invention, are discussed, e.g., in Sambrook, et al. (1989), Molecular Cloning, a Laboratory Manual, Cold Harbor Laboratory Press, Cold Spring Harbor, N.Y.; Ausubel et al. (1995). Current Protocols in Molecular Biology, N.Y., John Wiley & Sons; Davis et al. (1986), Basic Methods in Molecular Biology, Elsevier Sciences Publishing, Inc., New York; Hames et al. (1985), Nucleic Acid Hybridization, IL Press; Dracopoli et al. Current Protocols in Human Genetics, John Wiley & Sons, Inc.; and Coligan et al. Current Protocols in Protein Science, John Wiley & Sons, Inc. See, also, the Examples herein.

In a specific embodiment of this invention, the transgenic animal does not comprise additional transgenic sequences. This means that the transgenic LSD1 construct present in the transgenic animal is the only transgenic construct present in the animal of this invention. The transgenic animal preferably does not comprise further genetic germ line modifications other than that described hereinabove. The genetic background of the transgenic animal of the invention is 'wild type' except for the genetic modifications described hereinabove. This embodiment is preferred, as it allows studying the effects induced by LSD1 overexpression and of any methods interfering with the action of LSD1.

In another embodiment, the animal of the invention overexpresses one or more known oncogenes or protooncogens, or it comprises a knockout of one or more tumor suppressor genes. In yet another embodiment, the animal overexpresses one or more tumor suppressor genes.

*Method for identifying potential thereapeutic agents*

Another aspect of this invention is a method for identifying a compound which inhibits tumor growth, comprising (a) administering a test compound to a transgenic animal according to the present invention and (b) determining the effect of the test compound on the initiation, maintenance, or progression of cancer in said transgenic animal, thereby identifying a compound that inhibits tumor growth.

The method may comprise the steps (i) administering a test compound to a transgenic animal according to the present invention, (ii) administering the same test compound to a control animal, and (iii) determining the effect of the test compound on the transgenic animal as compared to said control animal. The control animal is preferably an animal of the same species as the transgenic animal. The control animal does not overexpress the polypeptide encoded by the transgenic nucleotide sequence of the test animal used in step (i) or it overexpresses it to a significantly lower degree (reduced by at least 25%, at least 50% or at least 75%), relative to the amount of polypeptide overexpressed in a given tissue of the transgenic test animal used in step (i). In a first embodiment, the control animal does not carry the transgenic nucleotide sequence present in the test animal of step (i). In another embodiment, the control animal is also carrying the same transgenic nucleotide sequence as the transgenic animal used in step (i) but there is no or only a weak overexpression. This can be achieved by the use of inducible transgene constructs, see *supra.*

The effect to be determined in step (iii) may be any change in any clinically or biologically relevant parameter including tumour mass, tumor size, presence of tumor markers, number of tumors, presence of metastases, survival rate and the like (relative to the control animal). The methods of analysis include but are not limited to determining the tumor mass, number of tumors, presence of metastases, tumor size, detecting tumor markers, histological methods and biochemical methods.

The test compound may be selected as a compound which inhibits tumor growth if there is a significant difference in at least one of the clinically or biologically relevant parameters tested, for example when the parameter (e.g. tumour mass, number of tumours, tumor size, tumor markers etc.) in the control animal is significantly reduced (e.g. by at least 10%, preferably by at least 25%, more preferably by at least 50%) relative to that of the transgenic test animal used in step (i).

The compounds used as test compounds may be inhibitors of LSD1.

*LSD1 Inhibitors*

The LSD1 inhibitor to be used in accordance with this invention is a compound capable of reducing the amount of LSD1 mRNA or LSD1 protein in a cell and/or inhibiting at least one function of the LSD1 gene or the LSD1 protein. These functions include (1) the ability of LSD1 to interact with androgen receptor, and (2) the catalytic activity of LSD1.

1. Compounds capable of inhibiting expression of LSD1

The LSD1 inhibitor to be used in accordance with this invention may be a compound capable of inhibiting expression of the LSD1 gene in a cell. Various methods for inhibiting expression of genes in a cell are known to one of skill in the art. For example, expression of certain genes may be inhibited by using interfering RNA and/or antisense nucleic acids. It is preferred according to the present invention that the LSD1 inhibitor is selected from siRNA, shRNA, miRNA and antisense nucleic acids.

a) Interfering RNA

Inhibitory double stranded nucleic acids (interfering nucleic acids, or siNAs) can also be used to inhibit gene expression, using conventional procedures. Preferably the inhibitory molecule is an short interfering RNA (siRNA) molecule. Typical methods to design, make and use interfering RNA molecules are described, e.g., in U.S. Pat. No. 6,506,559, U.S. Pat. No. 6,506,559; US Pat. publication 20030206887; and PCT publications WO99/07409, WO99/32619, WO 00/01846, WO 00/44914, WO00/44895, WO01/29058, WO01/36646, WO01/75164, WO01/92513, WO 01/29058, WO01/89304, WO01/90401, WO02/16620, and WO02/29858.

b) Antisense nucleic acids

Antisense nucleic acids may be used to inhibit the expression of the LSD1 gene in a cell. Methods and techniques for designing and preparing antisense nucleic acids are known *per se.* The skilled person can provide suitable antisense nucleic acids on the basis of information derived from the nucleotide sequence of LSD1. Suitable techniques are described, e.g., in Asubel et al. ed., chapter 26 (1994-2008).

2. Compounds capable of inhibiting the ability of LSD1 to interact with androgen receptor.

In another embodiment the invention, the LSD1 inhibitor is capable of inhibiting the interaction of LSD1 protein with the androgen receptor. Such inhibitors include antibodies specifically binding to the LSD1 protein. The antibodies are preferably monoclonal antibodies. Techniques for generating monoclonal antibodies specifically binding to specific proteins are known to those of skill in the art (see, e.g., Harlow and Lane, Antibodies: A Laboratory Manual, Cold Spring Harbor 1988. Cold Spring Harbor Laboratory; or ULLMANN'S Biotechnology and Biochemical Engineering, pp 443-455, Wiley-VCH 2007). It is also possible to use antibodies directed against androgen receptor.

Another class of agents that can be screened for possible use as drugs are "small molecules," also referred to herein as "compounds," which are isolated from natural sources or made synthetically. In general, such molecules may be identified from large libraries of natural products or synthetic (or semi-synthetic) extracts or chemical libraries according to methods known in the art. Those skilled in the field of drug discovery and development will understand that the precise source of test extracts or compounds is not critical to the methods of the invention. Accordingly, virtually any number of chemical extracts or compounds can be used in the methods described herein. The types of extracts or compounds that may be tested include plant, fungal, prokaryotic or eukaryotic cell or organism-based extracts, fermentation broths, and synthetic compounds including modifications of existing compounds. Numerous methods are also available for generating random or directed synthesis (e.g., semi-synthesis or total synthesis) of any number of chemical compounds, including, but not limited to, saccharides, lipids, peptides, polypeptides and nucleic acids and derivatives thereof. Synthetic compound libraries are commercially available.

Alternatively, libraries of natural compounds in the form of bacterial, fungal, plant, and animal extracts are commercially available from a number of sources. In addition, natural and synthetically produced libraries can be generated according to methods known in the art, e.g., by standard extraction and fractionation methods. Furthermore any library or compound may readily be modified using standard chemical, physical, or biochemical methods.

Another class of agents that can be screened are antibodies, in particular monoclonal antibodies. These include certain antibodies targeting receptors that are overexpressed in cancer cells.

One of skill in the art will appreciate that the LSD1 inhibitors can be used alone or in combination with other compounds and therapeutic regimens to inhibit tumorigensis.

An effective amount of the inhibitor will be determined by the existence, nature, and extent of any adverse side-effects that accompany the administration of the composition; the $LD_{50}$ of the composition; and the side-effects of the composition at various concentrations. Typically, the amount of the composition administered will range from about 0.01 to about 20 mg per kg, more typically about 0.05 to about 15 mg per kg, even more typically about 0.1 to about 10 mg per kg body weight.

The inhibitor can be administered, for example, by intravenous infusion, orally, intraperitoneally, or subcutaneously. Oral administration is the preferred method of administration. The formulations of compounds can be presented in unit-dose or multidose sealed containers, such as ampoules and vials.

The LSD1 inhibitors are typically formulated with a pharmaceutically acceptable carrier before administration to an individual or subject. Pharmaceutically acceptable carriers are determined, in part, by the particular composition being administered, as well as by the particular method used to administer the composition. Accordingly, there are a wide variety of suitable formulations of pharmaceutical compositions of the present invention (see, e.g., Remington's Pharmaceutical Sciences, 17th ed., 1989).

## Examples

### Example 1: Generation of the targeting vector

To generate the vector for ubiquitous expression of human Flag tagged LSD1 in transgenic mice the following cloning steps were made. Two copies of the core chicken ß-globin insulator element HS4 from the Gary Felsenfeld laboratory (pNI-CD) were cloned into pSL301 using KpnI (pSL301-HS4). The insulator element was than isolated from this vector with EcoRV and cloned into a blunted NotI site in pSL301-TG-P containing an $ARR_2Pb$ promoter, a rabbit β-globin intron and a SV40 poly A site (pSL301-HS4-TG-P). In parallel LSD1 was cloned from pCMX-Flag-Namo with EcoRV und blunted NheI into blunted BamHI and BglII sites from pBS-β-pA-HS4 containing two copies of the core chicken β-globin insulator element HS4 (pBS-Namo-HS4). The letter vector linearized with EcoRV and the insert from pSL301-HS4-TG-P digested with EcoRV and blunted XhoI were ligated to generate pBS-$ARR_2Pb$-AOF2. Than, pBS-$ARR_2Pb$-AOF2 digested with blunted XbaI to remove the $ARR_2Pb$ promoter was ligated to the insert of pROSA26 promoter (Zambrowicz et al., 1997; Kisseberth *et al.,* 1999) digested with blunted XbaI and SaiI to generate pBS-ROSA26-AOF2 (SEQ ID NO:6). The map of the targeting vector is shown in Figure 4. For microinjection the insert was relieved with MluI and BstXI.

### Example 2: Generation of transgenic mice

All animals were housed in the experimental unit of the pathogen free barrier facility of the Central Clinical Research of the Freiburg University Medical Center in accordance with institutional guidelines and approved by the regional board. Transgenic mice were generated by pronuclear injection into fertilized eggs of FVB (Taketo *et al.* 1991) mice using standard procedures (Hogan *et al.* 1994). Three independent founder lines were analyzed. Genotyping and specificity of transgene expression was verified by PCR and RT-PCR, respectively in all 3 independent lines in RNA extracts of different organs. The following primers were used for genotyping generating a 153bp fragment using standard PCR conditions: 5'-AATGCCTTCGAATTCAGCAC-3' (SEQ ID NO 8); 5'-CCTTGTCATCGTCGTCCTTG-3' (SEQ ID NO 9). The following primers were used for RT-PCR amplifying a 404bp fragment of Flag-tagged LSD1 using standard conditions with 3% DMSO: 5'-gactacaaggacgacgat-3' (SEQ ID NO 11); 5'-CCGCTCGAGTCAGCTTTCAT CCATCTCTCTG-3' (SEQ ID NO 11).

**Example 3: Detection off LSD 1 protein in various tissues of the transgenic mice**

293 cells were transfected with 5 μ g of pCMX-Flag-Namo. Protein extract was prepared 24 h after transfection using SC buffer containing protease inhibitors. Protein extracts from the indicated mouse tissues were dissolved in SC buffer containing protease inhibitors after homogenization in liquid nitrogen using a mortar and a pistil. Following pre-clearing with a 40 μl 1:1 slurry of GammaBind-Sepharose (Pharmacia), 2mg of mouse tissue protein supernatants were incubated for 2.5 h with M2 α -Flag antibody (Sigma). Beads were washed five times with WB (10 mM Tris-HCl pH 8.0, 250 mM NaCl, 0.5% NP-40, 0.1 μg/μl bovine serum albumin, 0.5 mM Pefabloc) and analysed on a 10% SDS gel. Western blots were decorated with M2 antibody. Secondary antibody and chemiluminescence procedures were performed according to the manufacturer (Amersham).

Human FLAG-tagged LSD1 protein could be detected in various tissues from Rosa26-LSD1 transgenic mice, see Figure 2.

**Example 4: Detection of tumors in the transgenic mice**

Adult mice were killed by cervical dislocation. Various organs were dissected and photographed. Specimen were embed in paraffin after fixation in 4% PFA over night, washing in PBS, dehydration in Ethanol and xylene. Sections of 6μm thickness were generated with a microtome. Sections were dehydrated and stained with eosin and hematoxylin using standard procedures.

Analysis revealed the development of various tumors in the adult Rosa26-LSD1 transgenic mice, see Figure 3. Macroscopic (upper left panel) and microscopic (upper right panel) photographs of a bronchial adenocarcinoma are shown (arrows). Macroscopic image of a section through a liver lobe (bottom left panel) with hepatocellular carcinoma (arrow) is shown. The bottom right panel depicts a section through a peritoneal lipoma stained by haematoxylin and eosin.

**References**

Chau, C. M., Deng, Z., Kang, H., Lieberman, P. M. (2008). Cell cycle association of the retinoblastoma protein Rb and the histone demethylase LSD1 with the Epstein-Barr virus latency promoter Cp. J. Virol. 82:3428-37.

Forneris, F., Binda, C., Battaglioli, E., Mattevi, A. (2008). LSD1: oxidative chemistry for multifaceted functions in chromatin regulation. Trends Biochem. Sci. 33:181-9.

Hogan, B., Constantini, F., Beddington, R. (1994). Manipulating the mouse embryo. Cold Spring Laboratory Press, Woodbury NY, 2nd edition.

Huang, J., Sengupta, R., Espejo, A.B., Lee, M.G., Dorsey, J.A., Richter, M., Opravil, S., Shiekhattar, R., Bedford, M.T., Jenuwein, T., Berger, S.L. (2007). p53 is regulated by the lysine demethylase LSD1. Nature 449:105-8.

Kahl, P., Gullotti, L., Heukamp, L. C., Wolf, S., Friedrichs, N., Vorreuther, R., Solleder, G., Bastian, P. J., Ellinger, J., Metzger, E., Schule R., Buettner R. (2006). Androgen receptor coactivators lysine-specific histone demethylase 1 and four and a half LIM domain protein 2 predict risk of prostate cancer recurrence. Cancer Res. 66:11341-7.

Kouzarides, T. (2007). Chromatin modifications and their function. Cell 128: 693-705.

Kisseberth, W. C., Brettingen, N. T., Lohse J. K., Sandgren, E.P. (1999). Ubiquitous expression of marker transgenes in mice and rats. Dev. Biol. 214:128-38.

Lee, M. G., Wynder, C., Cooch, N., Shiekhattar, R. (2005). An essential role for CoREST in nucleosomal histone 3 lysine 4 demethylation. Nature 437:432-5.

Metzger, E., Wissmann, M., Yin, N., Muller, J. M., Schneider, R., Peters, A. H., Günther, T., Buettner, R., Schule, R. (2005). LSD1 demethylates repressive histone marks to promote androgen-receptor-dependent transcription. Nature 437:436-9.

Sharifi, N., Gulley, J. L., Dahut, W. L. (2005). Androgen deprivation therapy for prostate cancer. JAMA 294: 238-44.

Shi, Y., Lan, F., Matson, C., Mulligan, P., Whetstine, J. R., Cole, P. A., Casero, R. A., Shi, Y. (2004). Histone demethylation mediated by the nuclear amine oxidase homolog LSD1. Cel 119:941-53.

Spannhoff, A., Heinke, R., Bauer, I., Trojer, P., Metzger, E., Gust, R., Schüle, R., Brosch, G., Sippl, W., Jung, M. (2007). Target-based approach to inhibitors of histone arginine methyltransferases. J. Med. Chem. 50:2319-25.

Taketo M., Schroeder A. C., Mobraaten L. E., Gunning K. B., Hanten G., Fox R. R., Roderick T. H., Stewart C. L., Lilly F., Hansen C. T., and Overbeek P. A. (1991) FVB/N: An inbred mouse strain preferable for transgenic analyses. Proc. Natl. Acad. Sci. USA 88, 2065-2069.

Wang, J., Scully, K., Zhu, X., Cai, L., Zhang, J., Prefontaine, G. G., Krones, A., Ohgi, K. A., Zhu, P., Garcia-Bassets, I., Liu, F., Taylor, H., Lozach, J., Jayes, F. L., Korach, K. S., Glass, C. K., Fu, X. D., Rosenfeld, M. G. (2007) Opposing LSD1 complexes function in developmental gene activation and repression programmes. Nature. 446:882-7.
Wissmann, M., Yin, N., Müller, J. M., Greschik, H., Fodor, B. D., Jenuwein, T., Vogler, C., Schneider, R., Günther, T., Buettner, R., Metzger E., Schüle R. (2007). Cooperative demethylation by JMJD2C and LSD1 promotes androgen receptor-dependent gene expression. Nat. Cell Biol. 9: 347-53.
Zambrowicz, B. P., Imamoto, A., Fiering, S., Herzenberg, L. A., Kerr, W. G., Soriano, P. (1997). Disruption of overlapping transcripts in the ROSA beta geo 26 gene trap strain leads to widespread expression of beta-galactosidase in mouse embryos and hematopoietic cells. Proc. Natl. Acad. Sci. USA. 94:3789-94.

## Claims

1. A non-human transgenic animal whose genome comprises a stably integrated transgenic nucleotide sequence encoding Lysine-specific Demethylase 1 (LSD1) operably linked to a promoter.

2. The transgenic animal of claim 1, wherein said LSD1 is human LSD1.

3. The transgenic animal of claim 1 or 2, wherein said LSD1 comprises at least amino acids 2 to 852 of the amino acid sequence as shown in SEQ ID NO:3.

4. The transgenic animal according to any one of the preceding claims, wherein said transgenic nucleotide sequence comprises nucleotides 4 to 2556 of the nucleotide sequence as shown in SEQ ID NO:1.

5. The transgenic animal according to any one of the preceding claims, wherein said LSD1 is overexpressed in the transgenic animal.

6. The transgenic animal of claim 5, wherein said overexpression is present at least in skeletal muscle, prostate tissue, testis and heart muscle.

7. The transgenic animal according to any one of the preceding claims, wherein said promoter is a Rosa26 promoter.

8. The transgenic animal according to any one of the preceding claims, wherein said animal develops at least one tumor during its life.

9. The transgenic animal according to claim 8, wherein said tumor is selected from lung tumors, hepatocellular carcinoma and peritoneal lipoma.

10. The transgenic animal according to any one of the preceding claims, which is a rodent, preferably a mouse.

11. A transgenic animal according to any one of the preceding claims wherein said animal does not comprise other transgenic nucleotide sequences.

12. A method for generating a non-human transgenic animal according to any one of the preceding claims, comprising transfecting a target animal with a vector molecule comprising an expression cassette wherein said expression cassette comprises a polynucleotide encoding LSD1 operably linked to a promoter.

13. A method for identifying a compound which inhibits tumor growth, comprising (a) administering a test compound to a transgenic animal according to any one of claims 1 to 11 and (b) determining the effect of the test compound on the initiation, maintenance, or progression of cancer in said transgenic animal, thereby identifying a compound that inhibits tumor growth.

14. A method according to claim 13, wherein said test compound is selected from the group consisting of LSD1 inhibitors, modulators of androgen receptor, modulators of p53, and modulators of Rb.

15. The use of the non-human transgenic animal according to any one of claims 1 to 11 I as an animal model for cancer.

**Figure 1**

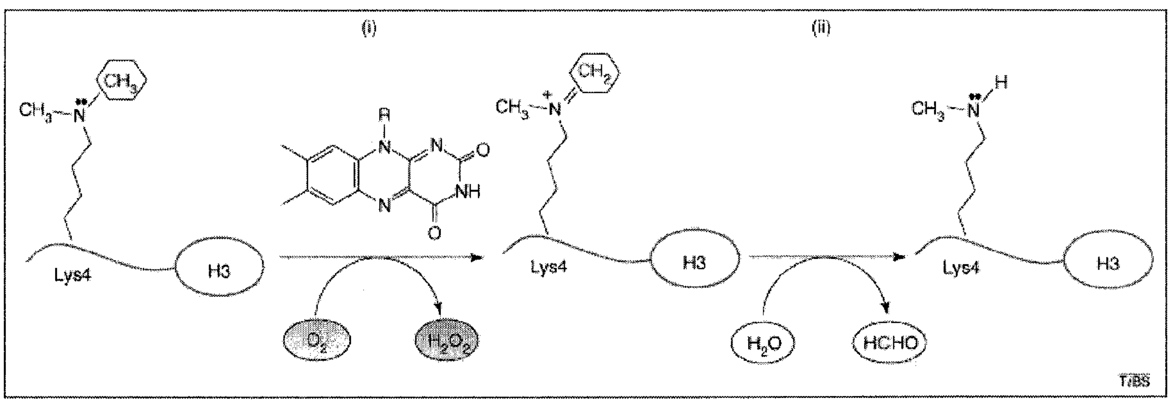

**Figure 2**

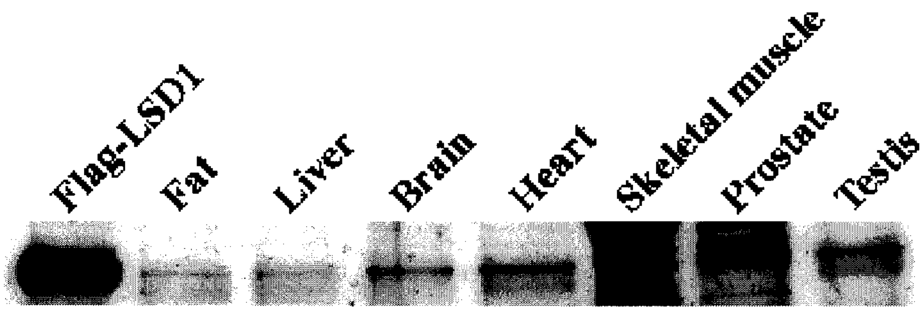
Flag-LSD1
Fat
Liver
Brain
Heart
Skeletal muscle
Prostate
Testis

**Figure 3**

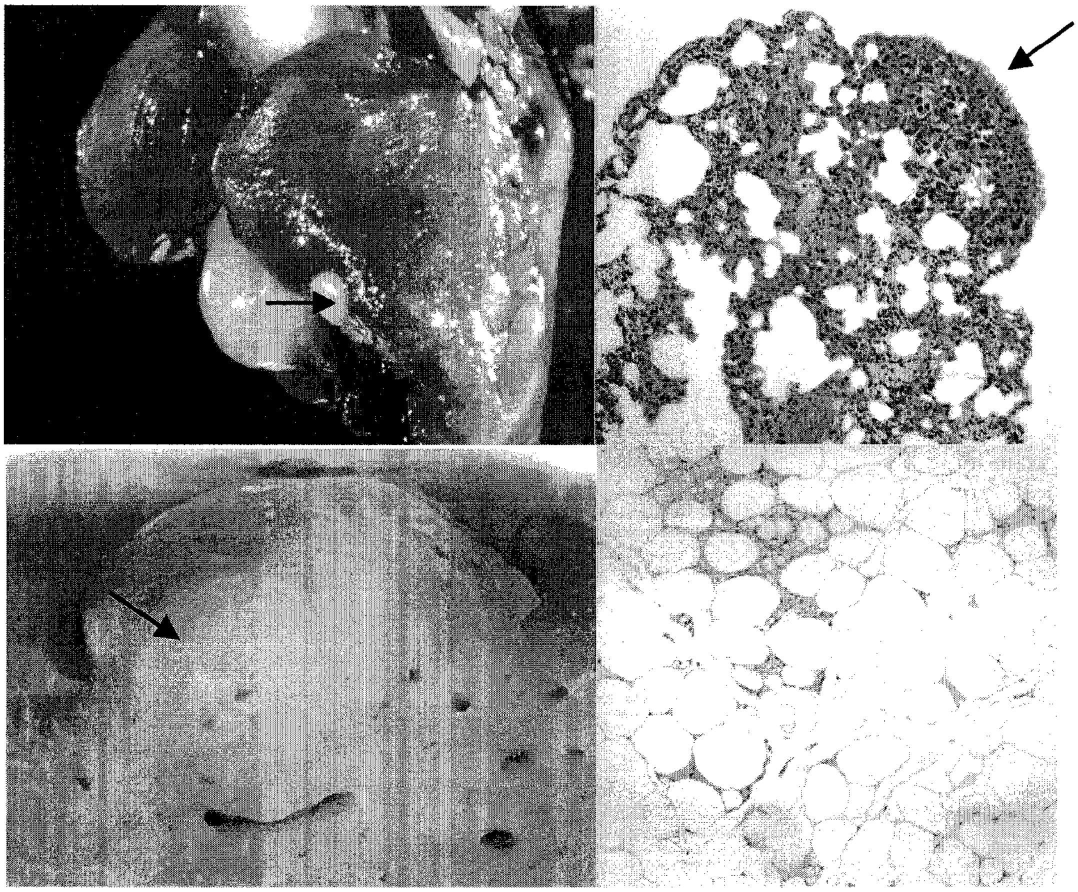

**Figure 4**

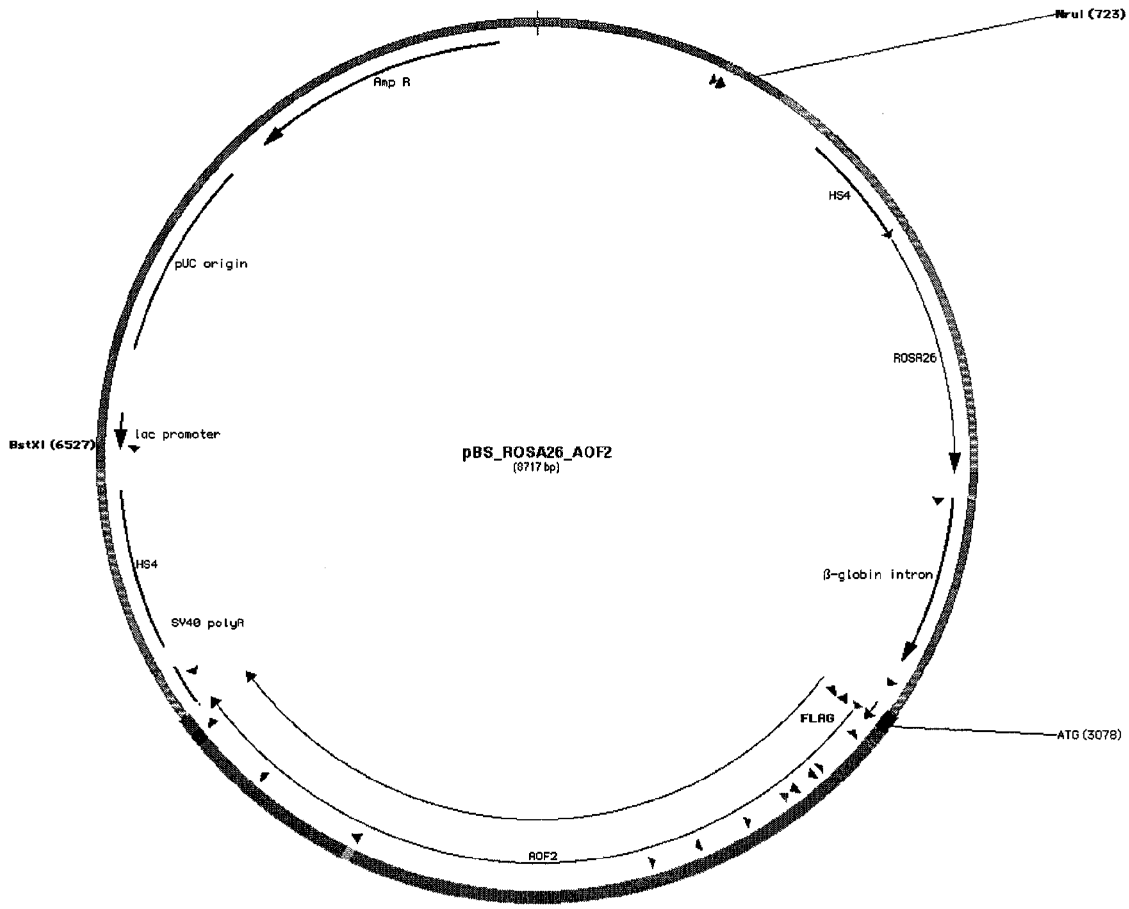
NruI (723)
Amp R
HS4
pUC origin
ROSA26
BstXI (6527)
lac promoter
pBS_ROSA26_AOF2
(8717 bp)
HS4
β-globin intron
SV40 polyA
FLAG
ATG (3078)
AOF2

Europäisches Patentamt
European Patent Office
Office européen des brevets

# EUROPEAN SEARCH REPORT

Application Number
EP 09 16 1995

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br><br><br><br><br><br><br><br><br><br><br>A | SCHULTE JOHANNES H ET AL: "Lysine-specific demethylase 1 is strongly expressed in poorly differentiated neuroblastoma: implications for therapy" CANCER RESEARCH, AMERICAN ASSOCIATION FOR CANCER RESEARCH, BALTIMORE, MD., US, vol. 69, no. 5, 17 February 2009 (2009-02-17), - 1 March 2009 (2009-03-01) pages 2065-2071, XP002541090 ISSN: 0008-5472<br>* the whole document *<br>----- | 1-12<br><br><br><br><br><br><br><br><br><br><br>13-15 | INV. C12N15/85 A01K67/027 |
| D,A | METZGER ERIC ET AL: "LSD1 demethylates repressive histone marks to promote androgen-receptor-dependent transcription" NATURE, NATURE PUBLISHING GROUP, LONDON, UK, vol. 437, no. 7057, 15 September 2005 (2005-09-15), pages 436-439,Suppl, XP002454571 ISSN: 0028-0836<br>* the whole document *<br>----- | 1 | TECHNICAL FIELDS SEARCHED (IPC) |
| A | KAHL PHILIP ET AL: "Androgen receptor coactivators lysine-specific histone demethylase 1 and four and a half LIM domain protein 2 predict risk of prostate cancer recurrence" CANCER RESEARCH 1 DEC 2006,, vol. 66, no. 23, 1 December 2006 (2006-12-01), pages 11341-11347, XP002554681<br>* the whole document *<br>-----<br>-/-- | 1 | C12N A01K |

The present search report has been drawn up for all claims

| Place of search | Date of completion of the search | Examiner |
|---|---|---|
| The Hague | 11 November 2009 | Chambonnet, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 09 16 1995

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2007/128982 A (CELLCENTRIC LTD [GB]; REIK WOLF [GB]; MORGAN HUGH [GB]; CHAN ANTHONY C) 15 November 2007 (2007-11-15) * claims 40-44 * | 1-12 | |
| A | METZGER E ET AL: "Histone demethylation and androgen-dependent transcription" CURRENT OPINION IN GENETICS & DEVELOPMENT, CURRENT BIOLOGY LTD, XX, vol. 16, no. 5, 1 October 2006 (2006-10-01), pages 513-517, XP024959760 ISSN: 0959-437X [retrieved on 2006-10-01] * the whole document * | 1 | |
| A | FORNERIS F, BINDA C, BATTAGLIOLI E, MATTEVI A.: "LSD1: oxidative chemistry for multifaceted functions in chromatin regulation." TRENDS BIOCHEM SCI., vol. 33, no. 4, 17 March 2008 (2008-03-17), pages 181-189, XP002554682 * the whole document * | 1 | TECHNICAL FIELDS SEARCHED (IPC) |
| A | WO 2006/071608 A (HARVARD COLLEGE [US]; SHI YANG [US]; SHI YUJIANG [US]) 6 July 2006 (2006-07-06) * the whole document * | 1,13,14 | |
| A | WO 2009/010121 A (UNIVERSITAETSKLINIKUM FREIBURG [DE]; SCHUELE ROLAND [DE]; METZGER ERIC) 22 January 2009 (2009-01-22) * claims * | 1 | |

-/--

The present search report has been drawn up for all claims

2

| Place of search | Date of completion of the search | Examiner |
|---|---|---|
| The Hague | 11 November 2009 | Chambonnet, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 16 1995

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | FORNERIS F, BATTAGLIOLI E, MATTEVI A, BINDA C.: "New roles of flavoproteins in molecular cell biology: histone demethylase LSD1 and chromatin." FEBS J., vol. 276, no. 16, 14 July 2009 (2009-07-14), - August 2009 (2009-08) pages 4304-4312, XP002554683<br>* the whole document *<br>----- | 1 | |
| | | | TECHNICAL FIELDS SEARCHED (IPC) |

The present search report has been drawn up for all claims

2

| Place of search | Date of completion of the search | Examiner |
|---|---|---|
| The Hague | 11 November 2009 | Chambonnet, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 16 1995

This annex lists the patent family members relating to the patent documents cited in the above-mentioned European search report.
The members are as contained in the European Patent Office EDP file on
The European Patent Office is in no way liable for these particulars which are merely given for the purpose of information.

11-11-2009

| Patent document cited in search report | Publication date | Patent family member(s) | Publication date |
|---|---|---|---|
| WO 2007128982 A | 15-11-2007 | NONE | |
| WO 2006071608 A | 06-07-2006 | AU 2005322312 A1 | 06-07-2006 |
| | | CA 2591717 A1 | 06-07-2006 |
| | | EP 1833981 A2 | 19-09-2007 |
| | | US 2009170796 A1 | 02-07-2009 |
| WO 2009010121 A | 22-01-2009 | EP 2017621 A1 | 21-01-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5378825 A
- WO 9418317 A
- WO 9502684 A
- WO 9505389 A
- US 6506559 B
- US 20030206887 A
- WO 9907409 A
- WO 9932619 A
- WO 0001846 A
- WO 0044914 A
- WO 0044895 A
- WO 0129058 A
- WO 0136646 A
- WO 0175164 A
- WO 0192513 A
- WO 0189304 A
- WO 0190401 A
- WO 0216620 A
- WO 0229858 A


### Non-patent literature cited in the description


- **Brinster et al.** *Proc. Natl. Acad. Sci. USA,* 1985, vol. 82, 4438
- **Jaenich.** *Proc. Natl. Acad. Sci. USA,* vol. 73, 1260
- **Evans et al.** *Nature,* 1981, vol. 292, 154
- **Tatusova et al.** *FEMS Microbiol. Lett.,* 1999, vol. 174, 247-250
- **Gossen, M. et al.** *Proc Natl Acad Sci USA,* 1992, vol. 89, 5547-51
- **Braselmann, S. et al.** *Proc Natl Acad Sci USA,* 1993, vol. 90, 1657-61
- **Weichselbaum, R R et al.** *Int J Radiation Oncology Biol Phys,* 1992, vol. 24, 565-67
- **Hallahan, D et al.** *Proc Natl Acad Sci USA,* 1991, vol. 88, 2152-20
- **Datta, R et al.** *Proc Natl Acad Sci USA,* 1992, vol. 89, 10149-53
- **Hallahan, D E et al.** *J Biol Chem,* 1993, vol. 268, 4903-07
- **Weichselbaum, R R et al.** *Intl J Radiation Oncology Bio. Phys,* 1994, vol. 30, 229-34
- **Hallahan, D E et al.** *Nature Med,* 1995, vol. 1, 786-91
- **Spencer, D. M. et al.** *Science,* 1993, vol. 262, 1019-1024
- **Travis.** *Science,* 1993, vol. 262, 989
- *Chem. & Eng. News,* 15 November 1993, 55-57
- **Weiss.** *Cell,* 1993, vol. 73, 209
- **Sambrook et al.** Molecular Cloning, a Laboratory Manual. Cold Harbor Laboratory Press, 1989
- **Ausubel et al.** Current Protocols in Molecular Biology. John Wiley & Sons, 1995
- **Davis et al.** Basic Methods in Molecular Biology. Elsevier Sciences Publishing, Inc, 1986
- **Hames et al.** Nucleic Acid Hybridization. IL Press, 1985
- **Dracopoli et al.** Current Protocols in Human Genetics. John Wiley & Sons, Inc,
- **Coligan et al.** Current Protocols in Protein Science. John Wiley & Sons, Inc
- **Harlow ; Lane.** Antibodies: A Laboratory Manual. Cold Spring Harbor Laboratory, 1988
- ULLMANN'S Biotechnology and Biochemical Engineering. Wiley-VCH, 2007, 443-455
- Remington's Pharmaceutical Sciences. 1989
- **Chau, C. M. ; Deng, Z. ; Kang, H. ; Lieberman, P. M.** Cell cycle association of the retinoblastoma protein Rb and the histone demethylase LSD1 with the Epstein-Barr virus latency promoter Cp. *J. Virol.,* 2008, vol. 82, 3428-37
- **Forneris, F. ; Binda, C. ; Battaglioli, E. ; Mattevi, A.** LSD1: oxidative chemistry for multifaceted functions in chromatin regulation. *Trends Biochem. Sci.,* 2008, vol. 33, 181-9
- **Hogan, B. ; Constantini, F. ; Beddington, R.** Manipulating the mouse embryo. Cold Spring Laboratory Press, 1994
- **Huang, J. ; Sengupta, R. ; Espejo, A.B. ; Lee, M.G. ; Dorsey, J.A. ; Richter, M. ; Opravil, S. ; Shiekhattar, R. ; Bedford, M.T. ; Jenuwein, T.** p53 is regulated by the lysine demethylase LSD1. *Nature,* 2007, vol. 449, 105-8
- **Kahl, P. ; Gullotti, L. ; Heukamp, L. C. ; Wolf, S. ; Friedrichs, N. ; Vorreuther, R. ; Solleder, G. ; Bastian, P. J. ; Ellinger, J. ; Metzger, E.** Androgen receptor coactivators lysine-specific histone demethylase 1 and four and a half LIM domain protein 2 predict risk of prostate cancer recurrence. *Cancer Res.,* 2006, vol. 66, 11341-7

- **Kouzarides, T.** Chromatin modifications and their function. *Cell,* 2007, vol. 128, 693-705
- **Kisseberth, W. C. ; Brettingen, N. T. ; Lohse J. K. ; Sandgren, E.P.** Ubiquitous expression of marker transgenes in mice and rats. *Dev. Biol.,* 1999, vol. 214, 128-38
- **Lee, M. G. ; Wynder, C. ; Cooch, N. ; Shiekhattar, R.** An essential role for CoREST in nucleosomal histone 3 lysine 4 demethylation. *Nature,* 2005, vol. 437, 432-5
- **Metzger, E. ; Wissmann, M. ; Yin, N. ; Muller, J. M. ; Schneider, R. ; Peters, A. H. ; Günther, T. ; Buettner, R. ; Schule, R.** LSD1 demethylates repressive histone marks to promote androgen-receptor-dependent transcription. *Nature,* 2005, vol. 437, 436-9
- **Sharifi, N. ; Gulley, J. L. ; Dahut, W. L.** Androgen deprivation therapy for prostate cancer. *JAMA,* 2005, vol. 294, 238-44
- **Shi, Y. ; Lan, F. ; Matson, C. ; Mulligan, P. ; Whetstine, J. R. ; Cole, P. A. ; Casero, R. A. ; Shi, Y.** Histone demethylation mediated by the nuclear amine oxidase homolog LSD1. *Cel,* 2004, vol. 119, 941-53
- **Spannhoff, A. ; Heinke, R. ; Bauer, I. ; Trojer, P. ; Metzger, E. ; Gust, R. ; Schüle, R. ; Brosch, G. ; Sippl, W. ; Jung, M.** Target-based approach to inhibitors of histone arginine methyltransferases. *J. Med. Chem.,* 2007, vol. 50, 2319-25
- **Taketo M. ; Schroeder A. C. ; Mobraaten L. E. ; Gunning K. B. ; Hanten G. ; Fox R. R. ; Roderick T. H. ; Stewart C. L. ; Lilly F. ; Hansen C. T.** FVB/N: An inbred mouse strain preferable for transgenic analyses. *Proc. Natl. Acad. Sci. USA,* 1991, vol. 88, 2065-2069
- **Wang, J. ; Scully, K. ; Zhu, X. ; Cai, L. ; Zhang, J. ; Prefontaine, G. G. ; Krones, A. ; Ohgi, K. A. ; Zhu, P. ; Garcia-Bassets, I.** Opposing LSD1 complexes function in developmental gene activation and repression programmes. *Nature,* 2007, vol. 446, 882-7
- **Wissmann, M. ; Yin, N. ; Müller, J. M. ; Greschik, H. ; Fodor, B. D. ; Jenuwein, T. ; Vogler, C. ; Schneider, R. ; Günther, T. ; Buettner, R.** Cooperative demethylation by JMJD2C and LSD1 promotes androgen receptor-dependent gene expression. *Nat. Cell Biol.,* 2007, vol. 9, 347-53
- **Zambrowicz, B. P. ; Imamoto, A. ; Fiering, S. ; Herzenberg, L. A. ; Kerr, W. G. ; Soriano, P.** Disruption of overlapping transcripts in the ROSA beta geo 26 gene trap strain leads to widespread expression of beta-galactosidase in mouse embryos and hematopoietic cells. *Proc. Natl. Acad. Sci. USA.,* 1997, vol. 94, 3789-94

SEQUENCE LISTING

<110> Universitaetsklinikum Freiburg

<120> Animal model for cancer

<130> LSD1

<160> 11

<170> PatentIn version 3.5

<210> 1
<211> 2559
<212> DNA
<213> homo sapiens

<400> 1

```
atgttatctg ggaagaaggc ggcagccgcg gcggcggcgg ctgcagcggc agcaaccggg      60
acggaggctg gccctgggac agcaggcggc tccgagaacg ggtctgaggt ggccgcgcag     120
cccgcgggcc tgtcgggccc agccgaggtc gggccggggg cggtggggga gcgcacaccc     180
cgcaagaaag agcctccgcg ggcctcgccc cccgggggcc tggcggaacc gccggggtcc     240
gcagggcctc aggccggccc tactgtcgtg cctgggtctg cgaccccccat ggaaactgga    300
atagcagaga ctccggaggg gcgtcggacc agccggcgca agcgggcgaa ggtagagtac     360
agagagatgg atgaaagctt ggccaacctc tcagaagatg agtattattc agaagaagag     420
agaaatgcca aagcagagaa ggaaaagaag cttcccccac cacccccctca agccccacct    480
gaggaagaaa atgaaagtga gcctgaagaa ccatcgggtg tggagggcgc agctttccag     540
agccgacttc ctcatgaccg gatgacttct caagaagcag cctgttttcc agatattatc     600
agtggaccac aacagaccca gaaggttttt cttttcatta gaaaccgcac actgcagttg     660
tggttggata atccaaagat tcagctgaca tttgaggcta ctctccaaca attagaagca     720
ccttataaca gtgatactgt gcttgtccac cgagttcaca gttatttaga gcgtcatggt     780
cttatcaact tcggcatcta taagaggata aaacccctac caactaaaaa gacaggaaag     840
gtaattatta taggctctgg ggtctcaggc ttggcagcag ctcgacagtt acaaagtttt     900
ggaatggatg tcacactttt ggaagccagg gatcgtgtgg gtggacgagt tgccacattt     960
cgcaaaggaa actatgtagc tgatcttgga gccatggtgg taacaggtct tggagggaat    1020
cctatggctg tggtcagcaa acaagtaaat atggaactgg ccaagatcaa gcaaaaatgc    1080
ccactttatg aagccaacgg acaagctgtt cctaaagaga aagatgaaat ggtagagcaa    1140
gagtttaacc ggttgctaga agctacatct taccttagtc atcaactaga cttcaatgtc    1200
ctcaataata agcctgtgtc ccttggccag gcattggaag ttgtcattca gttacaagag    1260
aagcatgtca aagatgagca gattgaacat tggaagaaga tagtgaaaac tcaggaagaa    1320
ttgaaagaac ttcttaataa gatggtaaat ttgaaagaga aaattaaaga actccatcag    1380
caatacaaag aagcatctga agtaaagcca cccagagata ttactgccga gttcttagtg    1440
aaaagcaaac acagggatct gaccgcccta tgcaaggaat atgatgaatt agctgaaaca    1500
caaggaaagc tagaagaaaa acttcaggag ttggaagcga atcccccaag tgatgtatat    1560
```

ctctcatcaa gagacagaca aatacttgat tggcattttg caaatcttga atttgctaat 1620

gccacacctc tctcaactct ctcccttaag cactgggatc aggatgatga ctttgagttc 1680

actggcagcc acctgacagt aaggaatggc tactcgtgtg tgcctgtggc tttagcagaa 1740

ggcctagaca ttaaactgaa tacagcagtg cgacaggttc gctacacggc ttcaggatgt 1800

gaagtgatag ctgtgaatac ccgctccacg agtcaaacct ttatttataa atgcgacgca 1860

gttctctgta cccttcccct gggtgtgctg aagcagcagc caccagccgt tcagtttgtg 1920

ccacctctcc ctgagtggaa aacatctgca gtccaaagga tgggatttgg caaccttaac 1980

aaggtggtgt tgtgttttga tcgggtgttc tgggatccaa gtgtcaattt gttcgggcat 2040

gttggcagta cgactgccag caggggtgag ctcttcctct tctggaacct ctataaagct 2100

ccaatactgt tggcactagt ggcaggagaa gctgctggta tcatggaaaa cataagtgac 2160

gatgtgattg ttggccgatg cctggccatt ctcaaaggga tttttggtag cagtgcagta 2220

cctcagccca aagaaactgt ggtgtctcgt tggcgtgctg atccctgggc tcggggctct 2280

tattcctatg ttgctgcagg atcatctgga aatgactatg atttaatggc tcagccaatc 2340

actcctggcc cctcgattcc aggtgcccca cagccgattc cacgactctt ctttgcggga 2400

gaacatacga tccgtaacta cccagccaca gtgcatggtg ctctgctgag tgggctgcga 2460

gaagcgggaa gaattgcaga ccagtttttg gggccatgt atacgctgcc tcgccaggcc 2520

acaccaggtg ttcctgcaca gcagtcccca agcatgtga 2559

<210> 2
<211> 3053
<212> DNA
<213> homo sapiens

<220>
<221> CDS
<222> (150)..(2708)

<400> 2
ggcgcggcgg gagcgcgctt ggcgcgtgcg tacgcgacgg cggttggcgg cgcgcgggca 60

gcgtgaagcg aggcgaggca aggcttttcg gacccacgga gcgacagagc gagcggcccc 120

```
tacggccgtc ggcggcccgg cggcccgag atg tta tct ggg aag aag gcg gca      173
                                Met Leu Ser Gly Lys Lys Ala Ala
                                1               5

gcc gcg gcg gcg gcg gct gca gcg gca gca acc ggg acg gag gct ggc      221
Ala Ala Ala Ala Ala Ala Ala Ala Ala Ala Thr Gly Thr Glu Ala Gly
    10                  15                  20

cct ggg aca gca ggc ggc tcc gag aac ggg tct gag gtg gcc gcg cag      269
Pro Gly Thr Ala Gly Gly Ser Glu Asn Gly Ser Glu Val Ala Ala Gln
25                  30                  35                  40

ccc gcg ggc ctg tcg ggc cca gcc gag gtc ggg ccg ggg gcg gtg ggg      317
Pro Ala Gly Leu Ser Gly Pro Ala Glu Val Gly Pro Gly Ala Val Gly
                45                  50                  55

gag cgc aca ccc cgc aag aaa gag cct ccg cgg gcc tcg ccc ccc ggg      365
Glu Arg Thr Pro Arg Lys Lys Glu Pro Pro Arg Ala Ser Pro Pro Gly
```

```
            60                      65                      70

ggc ctg gcg gaa ccg ccg ggg tcc gca ggg cct cag gcc ggc cct act       413
Gly Leu Ala Glu Pro Pro Gly Ser Ala Gly Pro Gln Ala Gly Pro Thr
        75                      80                      85

gtc gtg cct ggg tct gcg acc ccc atg gaa act gga ata gca gag act       461
Val Val Pro Gly Ser Ala Thr Pro Met Glu Thr Gly Ile Ala Glu Thr
    90                      95                      100

ccg gag ggg cgt cgg acc agc cgg cgc aag cgg gcg aag gta gag tac       509
Pro Glu Gly Arg Arg Thr Ser Arg Arg Lys Arg Ala Lys Val Glu Tyr
105                     110                     115                 120

aga gag atg gat gaa agc ttg gcc aac ctc tca gaa gat gag tat tat       557
Arg Glu Met Asp Glu Ser Leu Ala Asn Leu Ser Glu Asp Glu Tyr Tyr
                125                     130                 135

tca gaa gaa gag aga aat gcc aaa gca gag aag gaa aag aag ctt ccc       605
Ser Glu Glu Glu Arg Asn Ala Lys Ala Glu Lys Glu Lys Lys Leu Pro
            140                     145                     150

cca cca ccc cct caa gcc cca cct gag gaa gaa aat gaa agt gag cct       653
Pro Pro Pro Pro Gln Ala Pro Pro Glu Glu Glu Asn Glu Ser Glu Pro
        155                     160                     165

gaa gaa cca tcg ggt gtg gag ggc gca gct ttc cag agc cga ctt cct       701
Glu Glu Pro Ser Gly Val Glu Gly Ala Ala Phe Gln Ser Arg Leu Pro
    170                     175                     180

cat gac cgg atg act tct caa gaa gca gcc tgt ttt cca gat att atc       749
His Asp Arg Met Thr Ser Gln Glu Ala Ala Cys Phe Pro Asp Ile Ile
185                     190                     195                 200

agt gga cca caa cag acc cag aag gtt ttt ctt ttc att aga aac cgc       797
Ser Gly Pro Gln Gln Thr Gln Lys Val Phe Leu Phe Ile Arg Asn Arg
                205                     210                     215

aca ctg cag ttg tgg ttg gat aat cca aag att cag ctg aca ttt gag       845
Thr Leu Gln Leu Trp Leu Asp Asn Pro Lys Ile Gln Leu Thr Phe Glu
            220                     225                     230

gct act ctc caa caa tta gaa gca cct tat aac agt gat act gtg ctt       893
Ala Thr Leu Gln Gln Leu Glu Ala Pro Tyr Asn Ser Asp Thr Val Leu
        235                     240                     245

gtc cac cga gtt cac agt tat tta gag cgt cat ggt ctt atc aac ttc       941
Val His Arg Val His Ser Tyr Leu Glu Arg His Gly Leu Ile Asn Phe
    250                     255                     260

ggc atc tat aag agg ata aaa ccc cta cca act aaa aag aca gga aag       989
Gly Ile Tyr Lys Arg Ile Lys Pro Leu Pro Thr Lys Lys Thr Gly Lys
265                     270                     275                 280

gta att att ata ggc tct ggg gtc tca ggc ttg gca gca gct cga cag      1037
Val Ile Ile Ile Gly Ser Gly Val Ser Gly Leu Ala Ala Ala Arg Gln
                285                     290                     295

tta caa agt ttt gga atg gat gtc aca ctt ttg gaa gcc agg gat cgt      1085
Leu Gln Ser Phe Gly Met Asp Val Thr Leu Leu Glu Ala Arg Asp Arg
            300                     305                     310

gtg ggt gga cga gtt gcc aca ttt cgc aaa gga aac tat gta gct gat      1133
Val Gly Gly Arg Val Ala Thr Phe Arg Lys Gly Asn Tyr Val Ala Asp
        315                     320                     325

ctt gga gcc atg gtg gta aca ggt ctt gga ggg aat cct atg gct gtg      1181
Leu Gly Ala Met Val Val Thr Gly Leu Gly Gly Asn Pro Met Ala Val
    330                     335                     340
```

```
gtc agc aaa caa gta aat atg gaa ctg gcc aag atc aag caa aaa tgc      1229
Val Ser Lys Gln Val Asn Met Glu Leu Ala Lys Ile Lys Gln Lys Cys
345                 350                 355                 360

cca ctt tat gaa gcc aac gga caa gct gtt cct aaa gag aaa gat gaa      1277
Pro Leu Tyr Glu Ala Asn Gly Gln Ala Val Pro Lys Glu Lys Asp Glu
                365                 370                 375

atg gta gag caa gag ttt aac cgg ttg cta gaa gct aca tct tac ctt      1325
Met Val Glu Gln Glu Phe Asn Arg Leu Leu Glu Ala Thr Ser Tyr Leu
            380                 385                 390

agt cat caa cta gac ttc aat gtc ctc aat aat aag cct gtg tcc ctt      1373
Ser His Gln Leu Asp Phe Asn Val Leu Asn Asn Lys Pro Val Ser Leu
        395                 400                 405

ggc cag gca ttg gaa gtt gtc att cag tta caa gag aag cat gtc aaa      1421
Gly Gln Ala Leu Glu Val Val Ile Gln Leu Gln Glu Lys His Val Lys
    410                 415                 420

gat gag cag att gaa cat tgg aag aag ata gtg aaa act cag gaa gaa      1469
Asp Glu Gln Ile Glu His Trp Lys Lys Ile Val Lys Thr Gln Glu Glu
425                 430                 435                 440

ttg aaa gaa ctt ctt aat aag atg gta aat ttg aaa gag aaa att aaa      1517
Leu Lys Glu Leu Leu Asn Lys Met Val Asn Leu Lys Glu Lys Ile Lys
                445                 450                 455

gaa ctc cat cag caa tac aaa gaa gca tct gaa gta aag cca ccc aga      1565
Glu Leu His Gln Gln Tyr Lys Glu Ala Ser Glu Val Lys Pro Pro Arg
            460                 465                 470

gat att act gcc gag ttc tta gtg aaa agc aaa cac agg gat ctg acc      1613
Asp Ile Thr Ala Glu Phe Leu Val Lys Ser Lys His Arg Asp Leu Thr
        475                 480                 485

gcc cta tgc aag gaa tat gat gaa tta gct gaa aca caa gga aag cta      1661
Ala Leu Cys Lys Glu Tyr Asp Glu Leu Ala Glu Thr Gln Gly Lys Leu
    490                 495                 500

gaa gaa aaa ctt cag gag ttg gaa gcg aat ccc cca agt gat gta tat      1709
Glu Glu Lys Leu Gln Glu Leu Glu Ala Asn Pro Pro Ser Asp Val Tyr
505                 510                 515                 520

ctc tca tca aga gac aga caa ata ctt gat tgg cat ttt gca aat ctt      1757
Leu Ser Ser Arg Asp Arg Gln Ile Leu Asp Trp His Phe Ala Asn Leu
                525                 530                 535

gaa ttt gct aat gcc aca cct ctc tca act ctc tcc ctt aag cac tgg      1805
Glu Phe Ala Asn Ala Thr Pro Leu Ser Thr Leu Ser Leu Lys His Trp
            540                 545                 550

gat cag gat gat gac ttt gag ttc act ggc agc cac ctg aca gta agg      1853
Asp Gln Asp Asp Asp Phe Glu Phe Thr Gly Ser His Leu Thr Val Arg
        555                 560                 565

aat ggc tac tcg tgt gtg cct gtg gct tta gca gaa ggc cta gac att      1901
Asn Gly Tyr Ser Cys Val Pro Val Ala Leu Ala Glu Gly Leu Asp Ile
    570                 575                 580

aaa ctg aat aca gca gtg cga cag gtt cgc tac acg gct tca gga tgt      1949
Lys Leu Asn Thr Ala Val Arg Gln Val Arg Tyr Thr Ala Ser Gly Cys
585                 590                 595                 600

gaa gtg ata gct gtg aat acc cgc tcc acg agt caa acc ttt att tat      1997
Glu Val Ile Ala Val Asn Thr Arg Ser Thr Ser Gln Thr Phe Ile Tyr
                605                 610                 615
```

```
aaa tgc gac gca gtt ctc tgt acc ctt ccc ctg ggt gtg ctg aag cag      2045
Lys Cys Asp Ala Val Leu Cys Thr Leu Pro Leu Gly Val Leu Lys Gln
            620                 625                 630

cag cca cca gcc gtt cag ttt gtg cca cct ctc cct gag tgg aaa aca      2093
Gln Pro Pro Ala Val Gln Phe Val Pro Pro Leu Pro Glu Trp Lys Thr
        635                 640                 645

tct gca gtc caa agg atg gga ttt ggc aac ctt aac aag gtg gtg ttg      2141
Ser Ala Val Gln Arg Met Gly Phe Gly Asn Leu Asn Lys Val Val Leu
    650                 655                 660

tgt ttt gat cgg gtg ttc tgg gat cca agt gtc aat ttg ttc ggg cat      2189
Cys Phe Asp Arg Val Phe Trp Asp Pro Ser Val Asn Leu Phe Gly His
665                 670                 675                 680

gtt ggc agt acg act gcc agc agg ggt gag ctc ttc ctc ttc tgg aac      2237
Val Gly Ser Thr Thr Ala Ser Arg Gly Glu Leu Phe Leu Phe Trp Asn
                685                 690                 695

ctc tat aaa gct cca ata ctg ttg gca cta gtg gca gga gaa gct gct      2285
Leu Tyr Lys Ala Pro Ile Leu Leu Ala Leu Val Ala Gly Glu Ala Ala
            700                 705                 710

ggt atc atg gaa aac ata agt gac gat gtg att gtt ggc cga tgc ctg      2333
Gly Ile Met Glu Asn Ile Ser Asp Asp Val Ile Val Gly Arg Cys Leu
        715                 720                 725

gcc att ctc aaa ggg att ttt ggt agc agt gca gta cct cag ccc aaa      2381
Ala Ile Leu Lys Gly Ile Phe Gly Ser Ser Ala Val Pro Gln Pro Lys
    730                 735                 740

gaa act gtg gtg tct cgt tgg cgt gct gat ccc tgg gct cgg ggc tct      2429
Glu Thr Val Val Ser Arg Trp Arg Ala Asp Pro Trp Ala Arg Gly Ser
745                 750                 755                 760

tat tcc tat gtt gct gca gga tca tct gga aat gac tat gat tta atg      2477
Tyr Ser Tyr Val Ala Ala Gly Ser Ser Gly Asn Asp Tyr Asp Leu Met
                765                 770                 775

gct cag cca atc act cct ggc ccc tcg att cca ggt gcc cca cag ccg      2525
Ala Gln Pro Ile Thr Pro Gly Pro Ser Ile Pro Gly Ala Pro Gln Pro
            780                 785                 790

att cca cga ctc ttc ttt gcg gga gaa cat acg atc cgt aac tac cca      2573
Ile Pro Arg Leu Phe Phe Ala Gly Glu His Thr Ile Arg Asn Tyr Pro
        795                 800                 805

gcc aca gtg cat ggt gct ctg ctg agt ggg ctg cga gaa gcg gga aga      2621
Ala Thr Val His Gly Ala Leu Leu Ser Gly Leu Arg Glu Ala Gly Arg
    810                 815                 820

att gca gac cag ttt ttg ggg gcc atg tat acg ctg cct cgc cag gcc      2669
Ile Ala Asp Gln Phe Leu Gly Ala Met Tyr Thr Leu Pro Arg Gln Ala
825                 830                 835                 840

aca cca ggt gtt cct gca cag cag tcc cca agc atg tga gacagatgca       2718
Thr Pro Gly Val Pro Ala Gln Gln Ser Pro Ser Met
                845                 850

ttctaaggga agaggcccat gtgcctgttt ctgccatgta aggaaggctc ttctagcaat    2778

actagatccc actgagaaaa tccaccctgg catctgggct cctgatcagc tgatggagct    2838

cctgatttga caaaggagct tgcctccttt gaatgaccta gagcacaggg aggaacttgt    2898

ccattagttt ggaattgtgt tcttcgtaaa gactgaggca agcaagtgct gtgaaataac    2958

atcatcttag tcccttggtg tgtggggttt ttgttttttt tttatatttt gagaataaaa    3018
```

cttcatataa aattggcaaa aaaaaaaaaa aaaaa 3053

<210> 3
<211> 852
<212> PRT
<213> homo sapiens

<400> 3

Met Leu Ser Gly Lys Lys Ala Ala Ala Ala Ala Ala Ala Ala Ala Ala
1 5 10 15

Ala Ala Thr Gly Thr Glu Ala Gly Pro Gly Thr Ala Gly Gly Ser Glu
20 25 30

Asn Gly Ser Glu Val Ala Ala Gln Pro Ala Gly Leu Ser Gly Pro Ala
35 40 45

Glu Val Gly Pro Gly Ala Val Gly Glu Arg Thr Pro Arg Lys Lys Glu
50 55 60

Pro Pro Arg Ala Ser Pro Pro Gly Gly Leu Ala Glu Pro Pro Gly Ser
65 70 75 80

Ala Gly Pro Gln Ala Gly Pro Thr Val Val Pro Gly Ser Ala Thr Pro
85 90 95

Met Glu Thr Gly Ile Ala Glu Thr Pro Glu Gly Arg Arg Thr Ser Arg
100 105 110

Arg Lys Arg Ala Lys Val Glu Tyr Arg Glu Met Asp Glu Ser Leu Ala
115 120 125

Asn Leu Ser Glu Asp Glu Tyr Tyr Ser Glu Glu Glu Arg Asn Ala Lys
130 135 140

Ala Glu Lys Glu Lys Lys Leu Pro Pro Pro Pro Pro Gln Ala Pro Pro
145 150 155 160

Glu Glu Glu Asn Glu Ser Glu Pro Glu Glu Pro Ser Gly Val Glu Gly
165 170 175

Ala Ala Phe Gln Ser Arg Leu Pro His Asp Arg Met Thr Ser Gln Glu
180 185 190

Ala Ala Cys Phe Pro Asp Ile Ile Ser Gly Pro Gln Gln Thr Gln Lys
195 200 205

Val Phe Leu Phe Ile Arg Asn Arg Thr Leu Gln Leu Trp Leu Asp Asn
210 215 220

Pro Lys Ile Gln Leu Thr Phe Glu Ala Thr Leu Gln Gln Leu Glu Ala
225 230 235 240

```
Pro Tyr Asn Ser Asp Thr Val Leu Val His Arg Val His Ser Tyr Leu
                245                 250                 255

Glu Arg His Gly Leu Ile Asn Phe Gly Ile Tyr Lys Arg Ile Lys Pro
            260                 265                 270

Leu Pro Thr Lys Lys Thr Gly Lys Val Ile Ile Ile Gly Ser Gly Val
        275                 280                 285

Ser Gly Leu Ala Ala Ala Arg Gln Leu Gln Ser Phe Gly Met Asp Val
    290                 295                 300

Thr Leu Leu Glu Ala Arg Asp Arg Val Gly Gly Arg Val Ala Thr Phe
305                 310                 315                 320

Arg Lys Gly Asn Tyr Val Ala Asp Leu Gly Ala Met Val Val Thr Gly
                325                 330                 335

Leu Gly Gly Asn Pro Met Ala Val Val Ser Lys Gln Val Asn Met Glu
            340                 345                 350

Leu Ala Lys Ile Lys Gln Lys Cys Pro Leu Tyr Glu Ala Asn Gly Gln
        355                 360                 365

Ala Val Pro Lys Glu Lys Asp Glu Met Val Glu Gln Glu Phe Asn Arg
    370                 375                 380

Leu Leu Glu Ala Thr Ser Tyr Leu Ser His Gln Leu Asp Phe Asn Val
385                 390                 395                 400

Leu Asn Asn Lys Pro Val Ser Leu Gly Gln Ala Leu Glu Val Val Ile
                405                 410                 415

Gln Leu Gln Glu Lys His Val Lys Asp Glu Gln Ile Glu His Trp Lys
            420                 425                 430

Lys Ile Val Lys Thr Gln Glu Glu Leu Lys Glu Leu Leu Asn Lys Met
        435                 440                 445

Val Asn Leu Lys Glu Lys Ile Lys Glu Leu His Gln Gln Tyr Lys Glu
    450                 455                 460

Ala Ser Glu Val Lys Pro Pro Arg Asp Ile Thr Ala Glu Phe Leu Val
465                 470                 475                 480

Lys Ser Lys His Arg Asp Leu Thr Ala Leu Cys Lys Glu Tyr Asp Glu
                485                 490                 495

Leu Ala Glu Thr Gln Gly Lys Leu Glu Glu Lys Leu Gln Glu Leu Glu
            500                 505                 510
```

```
Ala Asn Pro Pro Ser Asp Val Tyr Leu Ser Ser Arg Asp Arg Gln Ile
        515                 520                 525

Leu Asp Trp His Phe Ala Asn Leu Glu Phe Ala Asn Ala Thr Pro Leu
    530                 535                 540

Ser Thr Leu Ser Leu Lys His Trp Asp Gln Asp Asp Asp Phe Glu Phe
545                 550                 555                 560

Thr Gly Ser His Leu Thr Val Arg Asn Gly Tyr Ser Cys Val Pro Val
                565                 570                 575

Ala Leu Ala Glu Gly Leu Asp Ile Lys Leu Asn Thr Ala Val Arg Gln
            580                 585                 590

Val Arg Tyr Thr Ala Ser Gly Cys Glu Val Ile Ala Val Asn Thr Arg
        595                 600                 605

Ser Thr Ser Gln Thr Phe Ile Tyr Lys Cys Asp Ala Val Leu Cys Thr
    610                 615                 620

Leu Pro Leu Gly Val Leu Lys Gln Gln Pro Pro Ala Val Gln Phe Val
625                 630                 635                 640

Pro Pro Leu Pro Glu Trp Lys Thr Ser Ala Val Gln Arg Met Gly Phe
                645                 650                 655

Gly Asn Leu Asn Lys Val Val Leu Cys Phe Asp Arg Val Phe Trp Asp
            660                 665                 670

Pro Ser Val Asn Leu Phe Gly His Val Gly Ser Thr Thr Ala Ser Arg
        675                 680                 685

Gly Glu Leu Phe Leu Phe Trp Asn Leu Tyr Lys Ala Pro Ile Leu Leu
    690                 695                 700

Ala Leu Val Ala Gly Glu Ala Ala Gly Ile Met Glu Asn Ile Ser Asp
705                 710                 715                 720

Asp Val Ile Val Gly Arg Cys Leu Ala Ile Leu Lys Gly Ile Phe Gly
                725                 730                 735

Ser Ser Ala Val Pro Gln Pro Lys Glu Thr Val Val Ser Arg Trp Arg
            740                 745                 750

Ala Asp Pro Trp Ala Arg Gly Ser Tyr Ser Tyr Val Ala Ala Gly Ser
        755                 760                 765

Ser Gly Asn Asp Tyr Asp Leu Met Ala Gln Pro Ile Thr Pro Gly Pro
    770                 775                 780
```

Ser Ile Pro Gly Ala Pro Gln Pro Ile Pro Arg Leu Phe Phe Ala Gly
785 790 795 800

Glu His Thr Ile Arg Asn Tyr Pro Ala Thr Val His Gly Ala Leu Leu
805 810 815

Ser Gly Leu Arg Glu Ala Gly Arg Ile Ala Asp Gln Phe Leu Gly Ala
820 825 830

Met Tyr Thr Leu Pro Arg Gln Ala Thr Pro Gly Val Pro Ala Gln Gln
835 840 845

Ser Pro Ser Met
850

<210> 4
<211> 3030
<212> DNA
<213> mus musculus

<220>
<221> CDS
<222> (139)..(2700)

<400> 4
gggcgcgtgc gcacgcgggg gtgtttggct tcgcacggag cgtgagaggt gcggggcgga 60

gaggcgcgag gcggctgcgg acccacggag cggcagaccg atcggcccct gcggcccgcg 120

gcggccaggc ggcccgag atg ttg tct ggg aag aag gcg gcg gcg gcg gca 171
Met Leu Ser Gly Lys Lys Ala Ala Ala Ala Ala
1 5 10

gcg gca gcg gcg gcg gcg gcg gct gct ggg acc gag gcc ggg tcc ggg 219
Ala Ala Ala Ala Ala Ala Ala Ala Ala Gly Thr Glu Ala Gly Ser Gly
15 20 25

gcg gcg ggc ggt gcc gag aac ggc tct gag gtg gcc gcg ccg ccc gcg 267
Ala Ala Gly Gly Ala Glu Asn Gly Ser Glu Val Ala Ala Pro Pro Ala
30 35 40

ggc ctg acg ggc ccc acc gac atg gct acg ggg gcg gcg ggc gag cgc 315
Gly Leu Thr Gly Pro Thr Asp Met Ala Thr Gly Ala Ala Gly Glu Arg
45 50 55

act ccc cga aag aag gag cct ccg cgg gcc tcg ccg ccc ggg ggc cta 363
Thr Pro Arg Lys Lys Glu Pro Pro Arg Ala Ser Pro Pro Gly Gly Leu
60 65 70 75

gcc gag ccg ccg ggg tct gct ggg ccc cag gcg ggg ccc aca gcc ggg 411
Ala Glu Pro Pro Gly Ser Ala Gly Pro Gln Ala Gly Pro Thr Ala Gly
80 85 90

ccc ggc tcc gcg acg ccc atg gag acc gga ata gcc gag acc ccg gag 459
Pro Gly Ser Ala Thr Pro Met Glu Thr Gly Ile Ala Glu Thr Pro Glu
95 100 105

ggc cga cgg acc agc cgg cgc aag cgg gcc aag gta gaa tac aga gaa 507
Gly Arg Arg Thr Ser Arg Arg Lys Arg Ala Lys Val Glu Tyr Arg Glu
110 115 120

atg gat gaa agc ttg gcc aac ctc tca gaa gat gaa tat tat tcg gaa 555
Met Asp Glu Ser Leu Ala Asn Leu Ser Glu Asp Glu Tyr Tyr Ser Glu

```
        125                 130                 135

gaa gaa aga aat gct aaa gca gag aag gaa aag aag ctt ccc cca cca        603
Glu Glu Arg Asn Ala Lys Ala Glu Lys Glu Lys Lys Leu Pro Pro Pro
140                 145                 150                 155

cct cct caa gcc cca cct gag gaa gaa aat gaa agt gag ccg gaa gag        651
Pro Pro Gln Ala Pro Pro Glu Glu Glu Asn Glu Ser Glu Pro Glu Glu
                160                 165                 170

ccg tct ggt gtg gag ggt gca gct ttt caa agc cga ctt ccc cat gac        699
Pro Ser Gly Val Glu Gly Ala Ala Phe Gln Ser Arg Leu Pro His Asp
            175                 180                 185

cga atg acc tct cag gaa gca gcc tgt ttc cca gac atc atc agt ggg        747
Arg Met Thr Ser Gln Glu Ala Ala Cys Phe Pro Asp Ile Ile Ser Gly
        190                 195                 200

cct cag cag aca cag aag gtt ttt ctg ttc atc agg aat cgc aca ttg        795
Pro Gln Gln Thr Gln Lys Val Phe Leu Phe Ile Arg Asn Arg Thr Leu
    205                 210                 215

cag tta tgg ctg gac aac cca aag atc cag ctg acg ttt gaa gcc act        843
Gln Leu Trp Leu Asp Asn Pro Lys Ile Gln Leu Thr Phe Glu Ala Thr
220                 225                 230                 235

ctc cag cag ctg gaa gcg cct tac aac agc gat act gtg ctt gtc cac        891
Leu Gln Gln Leu Glu Ala Pro Tyr Asn Ser Asp Thr Val Leu Val His
                240                 245                 250

cga gtt cac agt tac tta gag cgc cat ggt ctt atc aac ttc ggc atc        939
Arg Val His Ser Tyr Leu Glu Arg His Gly Leu Ile Asn Phe Gly Ile
            255                 260                 265

tac aag agg ata aaa ccc tta cca att aaa aag aca gga aag gtg att        987
Tyr Lys Arg Ile Lys Pro Leu Pro Ile Lys Lys Thr Gly Lys Val Ile
        270                 275                 280

att ata ggt tca ggt gtt tct ggc ttg gca gca gct cga cag cta cag       1035
Ile Ile Gly Ser Gly Val Ser Gly Leu Ala Ala Ala Arg Gln Leu Gln
    285                 290                 295

agt ttt ggg atg gat gtc aca ctt ctg gaa gcc agg gat cga gta ggt       1083
Ser Phe Gly Met Asp Val Thr Leu Leu Glu Ala Arg Asp Arg Val Gly
300                 305                 310                 315

gga cga gtt gct aca ttt cga aaa gga aac tat gta gct gat ctt ggc       1131
Gly Arg Val Ala Thr Phe Arg Lys Gly Asn Tyr Val Ala Asp Leu Gly
                320                 325                 330

gcc atg gtt gta aca ggt ctt gga ggg aat ccc atg gct gtc gtc agc       1179
Ala Met Val Val Thr Gly Leu Gly Gly Asn Pro Met Ala Val Val Ser
            335                 340                 345

aaa caa gta aat atg gaa ctg gcc aag atc aag caa aaa tgc cca ctt       1227
Lys Gln Val Asn Met Glu Leu Ala Lys Ile Lys Gln Lys Cys Pro Leu
        350                 355                 360

tat gaa gcc aat gga caa gct gtt cca aaa gaa aaa gat gaa atg gta       1275
Tyr Glu Ala Asn Gly Gln Ala Val Pro Lys Glu Lys Asp Glu Met Val
    365                 370                 375

gaa caa gaa ttt aac cgg ttg cta gaa gcc act tct tac ctt agt cac       1323
Glu Gln Glu Phe Asn Arg Leu Leu Glu Ala Thr Ser Tyr Leu Ser His
380                 385                 390                 395

cag tta gac ttc aac gtc ctc aat aat aaa cct gta tcc ctt ggc cag       1371
Gln Leu Asp Phe Asn Val Leu Asn Asn Lys Pro Val Ser Leu Gly Gln
                400                 405                 410
```

```
gca ttg gag gtt gtc att cag ctg caa gaa aag cat gtc aaa gat gag      1419
Ala Leu Glu Val Val Ile Gln Leu Gln Glu Lys His Val Lys Asp Glu
            415                 420                 425

cag att gaa cat tgg aag aag ata gtg aaa act cag gag gag ttg aaa      1467
Gln Ile Glu His Trp Lys Lys Ile Val Lys Thr Gln Glu Glu Leu Lys
        430                 435                 440

gag ctt ctt aat aag atg gta aat ttg aag gag aaa att aaa gag ctc      1515
Glu Leu Leu Asn Lys Met Val Asn Leu Lys Glu Lys Ile Lys Glu Leu
    445                 450                 455

cat cag caa tac aaa gaa gct tca gaa gtg aag ccg ccc aga gat atc      1563
His Gln Gln Tyr Lys Glu Ala Ser Glu Val Lys Pro Pro Arg Asp Ile
460                 465                 470                 475

aca gcc gag ttc ctg gtg aag agc aag cac agg gac ctg act gcc ctc      1611
Thr Ala Glu Phe Leu Val Lys Ser Lys His Arg Asp Leu Thr Ala Leu
                480                 485                 490

tgc aag gaa tat gat gaa tta gct gaa aca caa gga aag cta gaa gaa      1659
Cys Lys Glu Tyr Asp Glu Leu Ala Glu Thr Gln Gly Lys Leu Glu Glu
            495                 500                 505

aaa ctt caa gaa ttg gaa gcc aat ccc cca agt gat gta tac ctc tca      1707
Lys Leu Gln Glu Leu Glu Ala Asn Pro Pro Ser Asp Val Tyr Leu Ser
        510                 515                 520

tca aga gac aga caa ata ctt gac tgg cat ttt gca aat ctt gaa ttt      1755
Ser Arg Asp Arg Gln Ile Leu Asp Trp His Phe Ala Asn Leu Glu Phe
    525                 530                 535

gcc aac gcc aca cct ctc tct acc ctc tct ctt aaa cat tgg gat cag      1803
Ala Asn Ala Thr Pro Leu Ser Thr Leu Ser Leu Lys His Trp Asp Gln
540                 545                 550                 555

gat gat gac ttt gag ttt act gga agc cac ctg aca gta agg aat ggc      1851
Asp Asp Asp Phe Glu Phe Thr Gly Ser His Leu Thr Val Arg Asn Gly
                560                 565                 570

tac tca tgt gtg cct gtg gct tta gct gaa ggc ttg gac att aaa ctg      1899
Tyr Ser Cys Val Pro Val Ala Leu Ala Glu Gly Leu Asp Ile Lys Leu
            575                 580                 585

aac aca gca gtg cgg cag gtt cgc tac aca gcc tca gga tgt gaa gtg      1947
Asn Thr Ala Val Arg Gln Val Arg Tyr Thr Ala Ser Gly Cys Glu Val
        590                 595                 600

att gct gtg aac aca cgt tcc aca agt caa acc ttt att tat aag tgt      1995
Ile Ala Val Asn Thr Arg Ser Thr Ser Gln Thr Phe Ile Tyr Lys Cys
    605                 610                 615

gat gca gtt ctc tgt aca ctt cct ttg gga gtg ttg aag cag cag cca      2043
Asp Ala Val Leu Cys Thr Leu Pro Leu Gly Val Leu Lys Gln Gln Pro
620                 625                 630                 635

cca gct gtt cag ttt gtg cca cct ctt cct gag tgg aaa aca tct gca      2091
Pro Ala Val Gln Phe Val Pro Pro Leu Pro Glu Trp Lys Thr Ser Ala
                640                 645                 650

gtc caa agg atg gga ttt ggc aac ctt aac aag gtg gtg tta tgc ttt      2139
Val Gln Arg Met Gly Phe Gly Asn Leu Asn Lys Val Val Leu Cys Phe
            655                 660                 665

gac cgt gtg ttc tgg gac cca agt gtc aat ttg ttt ggg cac gtt ggc      2187
Asp Arg Val Phe Trp Asp Pro Ser Val Asn Leu Phe Gly His Val Gly
        670                 675                 680
```

```
agt aca act gct agc agg ggt gag ctc ttc ctc ttc tgg aac cta tat        2235
Ser Thr Thr Ala Ser Arg Gly Glu Leu Phe Leu Phe Trp Asn Leu Tyr
    685                 690                 695

aaa gct cca ata cta ttg gcc ctg gta gca gga gaa gct gct ggc att        2283
Lys Ala Pro Ile Leu Leu Ala Leu Val Ala Gly Glu Ala Ala Gly Ile
700                 705                 710                 715

atg gag aac att agt gat gat gtg att gtc ggc cgg tgc ctg gcc att        2331
Met Glu Asn Ile Ser Asp Asp Val Ile Val Gly Arg Cys Leu Ala Ile
                720                 725                 730

ctc aaa ggg att ttt ggc agc agt gca gtc cca cag ccc aag gaa act        2379
Leu Lys Gly Ile Phe Gly Ser Ser Ala Val Pro Gln Pro Lys Glu Thr
            735                 740                 745

gtg gta tct cgt tgg cgt gct gat ccg tgg gcc cgg ggc tcc tat tct        2427
Val Val Ser Arg Trp Arg Ala Asp Pro Trp Ala Arg Gly Ser Tyr Ser
        750                 755                 760

tat gtg gct gca gga tcc tct gga aat gac tat gat tta atg gct cag        2475
Tyr Val Ala Ala Gly Ser Ser Gly Asn Asp Tyr Asp Leu Met Ala Gln
    765                 770                 775

ccg atc act cct ggc ccc tca att cca ggt gcc cca cag cca atc cca        2523
Pro Ile Thr Pro Gly Pro Ser Ile Pro Gly Ala Pro Gln Pro Ile Pro
780                 785                 790                 795

aga ctc ttc ttt gct gga gaa cac aca atc cgg aac tac cca gct aca        2571
Arg Leu Phe Phe Ala Gly Glu His Thr Ile Arg Asn Tyr Pro Ala Thr
                800                 805                 810

gtc cat ggt gct ctg ttg agt ggg ctt cga gaa gca gga agg att gcc        2619
Val His Gly Ala Leu Leu Ser Gly Leu Arg Glu Ala Gly Arg Ile Ala
            815                 820                 825

gac cag ttt ttg gga gcc atg tac act ttg cct cgt cag gcc aca cca        2667
Asp Gln Phe Leu Gly Ala Met Tyr Thr Leu Pro Arg Gln Ala Thr Pro
        830                 835                 840

ggt gtc cct gca cag cag tcc cca agt atg tga gacagatggt tctgaacaga      2720
Gly Val Pro Ala Gln Gln Ser Pro Ser Met
    845                 850

gagatccaac ggcatgtcat ctgccacgta agcaagctct tctagcaata ctagatccta      2780

ctgagaaact ccatgtcatc agctactggg actcctagtt tgacagcaga ggctggctcc      2840

tttggctgac agcaacttac ccattgattt ggaagtacag ctccataaag actgctcgag      2900

aagcaagtgg tgtgagataa cctcttagtc tatggtgttt gtttgttttt gttttttttt      2960

aatatatttt gagaataaaa ctttaaaata attttatatg aaaatttatt tttaaaaaaa      3020

aaaaaaaaaa                                                             3030
```

<210> 5
<211> 853
<212> PRT
<213> mus musculus

<400> 5

```
Met Leu Ser Gly Lys Lys Ala Ala Ala Ala Ala Ala Ala Ala Ala Ala
1               5                   10                  15

Ala Ala Ala Ala Gly Thr Glu Ala Gly Ser Gly Ala Ala Gly Gly Ala
```

```
            20                  25                  30

Glu Asn Gly Ser Glu Val Ala Ala Pro Pro Ala Gly Leu Thr Gly Pro
        35                  40                  45

Thr Asp Met Ala Thr Gly Ala Ala Gly Glu Arg Thr Pro Arg Lys Lys
    50                  55                  60

Glu Pro Pro Arg Ala Ser Pro Pro Gly Gly Leu Ala Glu Pro Pro Gly
65                  70                  75                  80

Ser Ala Gly Pro Gln Ala Gly Pro Thr Ala Gly Pro Gly Ser Ala Thr
                85                  90                  95

Pro Met Glu Thr Gly Ile Ala Glu Thr Pro Glu Gly Arg Arg Thr Ser
            100                 105                 110

Arg Arg Lys Arg Ala Lys Val Glu Tyr Arg Glu Met Asp Glu Ser Leu
        115                 120                 125

Ala Asn Leu Ser Glu Asp Glu Tyr Tyr Ser Glu Glu Glu Arg Asn Ala
    130                 135                 140

Lys Ala Glu Lys Glu Lys Lys Leu Pro Pro Pro Pro Pro Gln Ala Pro
145                 150                 155                 160

Pro Glu Glu Glu Asn Glu Ser Glu Pro Glu Glu Pro Ser Gly Val Glu
                165                 170                 175

Gly Ala Ala Phe Gln Ser Arg Leu Pro His Asp Arg Met Thr Ser Gln
            180                 185                 190

Glu Ala Ala Cys Phe Pro Asp Ile Ile Ser Gly Pro Gln Gln Thr Gln
        195                 200                 205

Lys Val Phe Leu Phe Ile Arg Asn Arg Thr Leu Gln Leu Trp Leu Asp
    210                 215                 220

Asn Pro Lys Ile Gln Leu Thr Phe Glu Ala Thr Leu Gln Gln Leu Glu
225                 230                 235                 240

Ala Pro Tyr Asn Ser Asp Thr Val Leu Val His Arg Val His Ser Tyr
                245                 250                 255

Leu Glu Arg His Gly Leu Ile Asn Phe Gly Ile Tyr Lys Arg Ile Lys
            260                 265                 270

Pro Leu Pro Ile Lys Lys Thr Gly Lys Val Ile Ile Ile Gly Ser Gly
        275                 280                 285

Val Ser Gly Leu Ala Ala Ala Arg Gln Leu Gln Ser Phe Gly Met Asp
    290                 295                 300
```

Val Thr Leu Leu Glu Ala Arg Asp Arg Val Gly Gly Arg Val Ala Thr
305 310 315 320

Phe Arg Lys Gly Asn Tyr Val Ala Asp Leu Gly Ala Met Val Val Thr
325 330 335

Gly Leu Gly Gly Asn Pro Met Ala Val Val Ser Lys Gln Val Asn Met
340 345 350

Glu Leu Ala Lys Ile Lys Gln Lys Cys Pro Leu Tyr Glu Ala Asn Gly
355 360 365

Gln Ala Val Pro Lys Glu Lys Asp Glu Met Val Glu Gln Glu Phe Asn
370 375 380

Arg Leu Leu Glu Ala Thr Ser Tyr Leu Ser His Gln Leu Asp Phe Asn
385 390 395 400

Val Leu Asn Asn Lys Pro Val Ser Leu Gly Gln Ala Leu Glu Val Val
405 410 415

Ile Gln Leu Gln Glu Lys His Val Lys Asp Glu Gln Ile Glu His Trp
420 425 430

Lys Lys Ile Val Lys Thr Gln Glu Glu Leu Lys Glu Leu Leu Asn Lys
435 440 445

Met Val Asn Leu Lys Glu Lys Ile Lys Glu Leu His Gln Gln Tyr Lys
450 455 460

Glu Ala Ser Glu Val Lys Pro Pro Arg Asp Ile Thr Ala Glu Phe Leu
465 470 475 480

Val Lys Ser Lys His Arg Asp Leu Thr Ala Leu Cys Lys Glu Tyr Asp
485 490 495

Glu Leu Ala Glu Thr Gln Gly Lys Leu Glu Glu Lys Leu Gln Glu Leu
500 505 510

Glu Ala Asn Pro Pro Ser Asp Val Tyr Leu Ser Ser Arg Asp Arg Gln
515 520 525

Ile Leu Asp Trp His Phe Ala Asn Leu Glu Phe Ala Asn Ala Thr Pro
530 535 540

Leu Ser Thr Leu Ser Leu Lys His Trp Asp Gln Asp Asp Asp Phe Glu
545 550 555 560

Phe Thr Gly Ser His Leu Thr Val Arg Asn Gly Tyr Ser Cys Val Pro
565 570 575

Val Ala Leu Ala Glu Gly Leu Asp Ile Lys Leu Asn Thr Ala Val Arg
580 585 590

Gln Val Arg Tyr Thr Ala Ser Gly Cys Glu Val Ile Ala Val Asn Thr
595 600 605

Arg Ser Thr Ser Gln Thr Phe Ile Tyr Lys Cys Asp Ala Val Leu Cys
610 615 620

Thr Leu Pro Leu Gly Val Leu Lys Gln Gln Pro Pro Ala Val Gln Phe
625 630 635 640

Val Pro Pro Leu Pro Glu Trp Lys Thr Ser Ala Val Gln Arg Met Gly
645 650 655

Phe Gly Asn Leu Asn Lys Val Val Leu Cys Phe Asp Arg Val Phe Trp
660 665 670

Asp Pro Ser Val Asn Leu Phe Gly His Val Gly Ser Thr Thr Ala Ser
675 680 685

Arg Gly Glu Leu Phe Leu Phe Trp Asn Leu Tyr Lys Ala Pro Ile Leu
690 695 700

Leu Ala Leu Val Ala Gly Glu Ala Ala Gly Ile Met Glu Asn Ile Ser
705 710 715 720

Asp Asp Val Ile Val Gly Arg Cys Leu Ala Ile Leu Lys Gly Ile Phe
725 730 735

Gly Ser Ser Ala Val Pro Gln Pro Lys Glu Thr Val Val Ser Arg Trp
740 745 750

Arg Ala Asp Pro Trp Ala Arg Gly Ser Tyr Ser Tyr Val Ala Ala Gly
755 760 765

Ser Ser Gly Asn Asp Tyr Asp Leu Met Ala Gln Pro Ile Thr Pro Gly
770 775 780

Pro Ser Ile Pro Gly Ala Pro Gln Pro Ile Pro Arg Leu Phe Phe Ala
785 790 795 800

Gly Glu His Thr Ile Arg Asn Tyr Pro Ala Thr Val His Gly Ala Leu
805 810 815

Leu Ser Gly Leu Arg Glu Ala Gly Arg Ile Ala Asp Gln Phe Leu Gly
820 825 830

Ala Met Tyr Thr Leu Pro Arg Gln Ala Thr Pro Gly Val Pro Ala Gln
835 840 845

```
Gln Ser Pro Ser Met
    850


<210>  6
<211>  8717
<212>  DNA
<213>  artificial sequence

<220>
<223>  pBS-ROSA26-AOF2


<220>
<221>  promoter
<222>  (625)..(645)
<223>  T7 promoter

<220>
<221>  misc_feature
<222>  (1018)..(1381)
<223>  HS4 insulator

<220>
<221>  promoter
<222>  (1414)..(2225)
<223>  ROSA26 promoter

<220>
<221>  Intron
<222>  (2311)..(2878)
<223>  rabbit beta-globin intron

<220>
<221>  misc_feature
<222>  (3079)..(3106)
<223>  sequence encoding FLAG tag

<220>
<221>  CDS
<222>  (3079)..(5667)

<220>
<221>  misc_feature
<222>  (3112)..(5667)
<223>  seqence encoding human LSD1

<220>
<221>  polyA_site
<222>  (5686)..(5819)
<223>  SV40 poly A site

<220>
<221>  misc_feature
<222>  (5896)..(6434)
<223>  HS4 insulator

<220>
<221>  misc_feature
<222>  (6914)..(7581)
<223>  pUC origin

<220>
<221>  misc_feature
<222>  (8589)..(7729)
<223>  sequence encoding beta-lactamase

<400>  6
ctaaattgta agcgttaata ttttgttaaa attcgcgtta aatttttgtt aaatcagctc        60
```

```
atttttttaac caataggccg aaatcggcaa aatcccttat aaatcaaaag aatagaccga      120
gatagggttg agtgttgttc cagtttggaa caagagtcca ctattaaaga acgtggactc      180
caacgtcaaa gggcgaaaaa ccgtctatca gggcgatggc ccactacgtg aaccatcacc      240
ctaatcaagt tttttggggt cgaggtgccg taaagcacta aatcggaacc ctaaagggag      300
cccccgattt agagcttgac ggggaaagcc ggcgaacgtg gcgagaaagg aagggaagaa      360
agcgaaagga gcgggcgcta gggcgctggc aagtgtagcg gtcacgctgc gcgtaaccac      420
cacacccgcc gcgcttaatg cgccgctaca gggcgcgtcc cattcgccat tcaggctgcg      480
caactgttgg gaagggcgat cggtgcgggc ctcttcgcta ttacgccagc tggcgaaagg      540
gggatgtgct gcaaggcgat taagttgggt aacgccaggg ttttcccagt cacgacgttg      600
taaaacgacg gccagtgagc gcgcgtaata cgactcacta tagggcgaat tgggtaccgg      660
gccccccctc gaggtcgacg gtatcgataa gcttgattcg agctctgtac atgtccgcgg      720
tcgcgacgta cgcgtatcga tggcgccagc tgcaggcggc cgccatatgc atcctaggcc      780
tattaatatt ccggagtata cgtagccggc taacgttaac aaccggtacc gagttggcgc      840
gcctgggagc tcacggggac agcccccccc caaagccccc agggatgtaa ttacgtccct      900
cccccgctag ggggcagcag cgagccgccc ggggctccgc tccggtccgg cgctcccccc      960
gcatccccga gccggcagcg tgcggggaca gcccgggcac ggggaaggtg gcacgggatc     1020
gctttcctct gaacgcttct cgctgctctt tgagcctgca gacacctggg gggatacggg     1080
gaaaaagctt taggctgaaa gagagattta gaatgacggg cgcgcctggg agctcacggg     1140
gacagccccc ccccaaagcc cccagggatg taattacgtc cctcccccgc tagggggcag     1200
cagcgagccg cccggggctc cgctccggtc cggcgctccc cccgcatccc cgagccggca     1260
gcgtgcgggg acagcccggg cacggggaag gtggcacggg atcgctttcc tctgaacgct     1320
tctcgctgct ctttgagcct gcagacacct gggggggatac ggggaaaaag ctgggcgcgc     1380
caattaaccc tcactaaagg gggtacctct agtcgactag atgaaggaga gcctttctct     1440
ctgggcaaga gcggtgcaat ggtgtgtaaa ggtagctgag aagacgaaaa gggcaagcat     1500
cttcctgcta ccaggctggg gaggcccagg cccacgaccc cgaggagagg gaacgcaggg     1560
agactgaggt gacccttctt tcccccgggg cccggtcgtg tggttcggtg tctcttttct     1620
gttggaccct taccttgacc caggcgctgc cggggcctgg gcccgggctg cggcgcacgg     1680
cactcccggg aggcagcgag actcgagtta ggcccaacgc ggcgccacgg cgtttcctgg     1740
ccgggaatgg cccgtacccg tgaggtgggg gtgggggggca gaaaaggcgg agcgagcccg     1800
aggcggggag ggggagggcc aggggcggag gggccggca ctactgtgtt ggcggactgg     1860
cgggactagg gctgcgtgag tctctgagcg caggcgggcg gcggccgccc ctcccccggc     1920
ggcggcagcg gcggcagcgg cggcagctca ctcagcccgc tgcccgagcg gaaacgccac     1980
tgaccgcacg gggattccca gtgccggcgc caggggcacg cgggacacgc cccctcccgc     2040
cgcgccattg gcctctccgc ccaccgcccc acacttattg gccggtgcgc cgccaatcag     2100
```

```
cggaggctgc cggggccgcc taaagaagag gctgtgcttt ggggctccgg ctcctcagag      2160
agcctcggct aggtagggga tcgggactct ggcgggaggg cggcttggtg cgtttgcggg      2220
gatccactag ttctagaact atagctagca tgcgcaaatt taaagcgctg atatcgatcg      2280
cgcgcagatc ctaagaactt ccaggggagg tttggggacc cttgattgtt ctttcttttt      2340
cgctattgta aaattcatgt tatatggagg gggcaaagtt ttcagggtgt tgtttagaat      2400
gggaagatgt cccttgtatc accatggacc ctcatgataa ttttgtttct ttcactttct      2460
actctgttga caaccattgt ctcctcttat tttcttttca ttttctgtaa ctttttcgtt      2520
aaactttagc ttgcatttgt aacgaatttt taaattcact tttgtttatt tgtcagattg      2580
taagtacttt ctctaatcac ttttttttca aggcaatcag ggtatattat attgtacttc      2640
agcacagttt tagagaacaa ttgttataat taaatgataa ggtagaatat ttctgcatat      2700
aaattctggc tggcgtggaa atattcttat tggtagaaac aactacaccc tggtcatcat      2760
cctgcctttc tctttatggt tacaatgata tacactgttt gagatgagga taaaatactc      2820
tgagtccaaa ccgggcccct ctgctaacca tgttcatgcc ttcttctctt tcctacagct      2880
cctgggcaac gtgctggtta tgtgctgtct catcaaatgg caaagaattc atggctggtg      2940
accacgtcgt ggaatgcctt cgaattcagc acctgcacat gggacgtcga cctgaggtaa      3000
ttataacccg ggccctatat atggatccag atcgatcatc aggatcggta ccgggccccc      3060
cctcgagaag cttccacc atg gac tac aag gac gac gat gac aag gaa ttc        3111
                    Met Asp Tyr Lys Asp Asp Asp Asp Lys Glu Phe
                    1               5                   10

tta tct ggg aag aag gcg gca gcc gcg gcg gcg gcg gct gca gcg gca        3159
Leu Ser Gly Lys Lys Ala Ala Ala Ala Ala Ala Ala Ala Ala Ala Ala
            15                  20                  25

gca acc ggg acg gag gct ggc cct ggg aca gca ggc ggc tcc gag aac        3207
Ala Thr Gly Thr Glu Ala Gly Pro Gly Thr Ala Gly Gly Ser Glu Asn
        30                  35                  40

ggg tct gag gtg gcc gcg cag ccc gcg ggc ctg tcg ggc cca gcc gag        3255
Gly Ser Glu Val Ala Ala Gln Pro Ala Gly Leu Ser Gly Pro Ala Glu
    45                  50                  55

gtc ggg ccg ggg gcg gtg ggg gag cgc aca ccc cgc aag aaa gag cct        3303
Val Gly Pro Gly Ala Val Gly Glu Arg Thr Pro Arg Lys Lys Glu Pro
60                  65                  70                  75

ccg cgg gcc tcg ccc ccc ggg ggc ctg gcg gaa ccg ccg ggg tcc gca        3351
Pro Arg Ala Ser Pro Pro Gly Gly Leu Ala Glu Pro Pro Gly Ser Ala
                80                  85                  90

ggg cct cag gcc ggc cct act gtc gtg cct ggg tct gcg acc ccc atg        3399
Gly Pro Gln Ala Gly Pro Thr Val Val Pro Gly Ser Ala Thr Pro Met
            95                  100                 105

gaa act gga ata gca gag act ccg gag ggg cgt cgg acc agc cgg cgc        3447
Glu Thr Gly Ile Ala Glu Thr Pro Glu Gly Arg Arg Thr Ser Arg Arg
        110                 115                 120

aag cgg gcg aag gta gag tac aga gag atg gat gaa agc ttg gcc aac        3495
Lys Arg Ala Lys Val Glu Tyr Arg Glu Met Asp Glu Ser Leu Ala Asn
    125                 130                 135

ctc tca gaa gat gag tat tat tca gaa gaa gag aga aat gcc aaa gca        3543
```

```
Leu Ser Glu Asp Glu Tyr Tyr Ser Glu Glu Glu Arg Asn Ala Lys Ala
140                 145                 150                 155

gag aag gaa aag aag ctt ccc cca cca ccc cct caa gcc cca cct gag        3591
Glu Lys Glu Lys Lys Leu Pro Pro Pro Pro Pro Gln Ala Pro Pro Glu
                160                 165                 170

gaa gaa aat gaa agt gag cct gaa gaa cca tcg ggt gtg gag ggc gca        3639
Glu Glu Asn Glu Ser Glu Pro Glu Glu Pro Ser Gly Val Glu Gly Ala
            175                 180                 185

gct ttc cag agc cga ctt cct cat gac cgg atg act tct caa gaa gca        3687
Ala Phe Gln Ser Arg Leu Pro His Asp Arg Met Thr Ser Gln Glu Ala
        190                 195                 200

gcc tgt ttt cca gat att atc agt gga cca caa cag acc cag aag gtt        3735
Ala Cys Phe Pro Asp Ile Ile Ser Gly Pro Gln Gln Thr Gln Lys Val
    205                 210                 215

ttt ctt ttc att aga aac cgc aca ctg cag ttg tgg ttg gat aat cca        3783
Phe Leu Phe Ile Arg Asn Arg Thr Leu Gln Leu Trp Leu Asp Asn Pro
220                 225                 230                 235

aag att cag ctg aca ttt gag gct act ctc caa caa tta gaa gca cct        3831
Lys Ile Gln Leu Thr Phe Glu Ala Thr Leu Gln Gln Leu Glu Ala Pro
                240                 245                 250

tat aac agt gat act gtg ctt gtc cac cga gtt cac agt tat tta gag        3879
Tyr Asn Ser Asp Thr Val Leu Val His Arg Val His Ser Tyr Leu Glu
            255                 260                 265

cgt cat ggt ctt atc aac ttc ggc atc tat aag agg ata aaa ccc cta        3927
Arg His Gly Leu Ile Asn Phe Gly Ile Tyr Lys Arg Ile Lys Pro Leu
        270                 275                 280

cca act aaa aag aca gga aag gta att att ata ggc tct ggg gtc tca        3975
Pro Thr Lys Lys Thr Gly Lys Val Ile Ile Ile Gly Ser Gly Val Ser
    285                 290                 295

ggc ttg gca gca gct cga cag tta caa agt ttt gga atg gat gtc aca        4023
Gly Leu Ala Ala Ala Arg Gln Leu Gln Ser Phe Gly Met Asp Val Thr
300                 305                 310                 315

ctt ttg gaa gcc agg gat cgt gtg ggt gga cga gtt gcc aca ttt cgc        4071
Leu Leu Glu Ala Arg Asp Arg Val Gly Gly Arg Val Ala Thr Phe Arg
                320                 325                 330

aaa gga aac tat gta gct gat ctt gga gcc atg gtg gta aca ggt ctt        4119
Lys Gly Asn Tyr Val Ala Asp Leu Gly Ala Met Val Val Thr Gly Leu
            335                 340                 345

gga ggg aat cct atg gct gtg gtc agc aaa caa gta aat atg gaa ctg        4167
Gly Gly Asn Pro Met Ala Val Val Ser Lys Gln Val Asn Met Glu Leu
        350                 355                 360

gcc aag atc aag caa aaa tgc cca ctt tat gaa gcc aac gga caa gct        4215
Ala Lys Ile Lys Gln Lys Cys Pro Leu Tyr Glu Ala Asn Gly Gln Ala
    365                 370                 375

gtt cct aaa gag aaa gat gaa atg gta gag caa gag ttt aac cgg ttg        4263
Val Pro Lys Glu Lys Asp Glu Met Val Glu Gln Glu Phe Asn Arg Leu
380                 385                 390                 395

cta gaa gct aca tct tac ctt agt cat caa cta gac ttc aat gtc ctc        4311
Leu Glu Ala Thr Ser Tyr Leu Ser His Gln Leu Asp Phe Asn Val Leu
                400                 405                 410

aat aat aag cct gtg tcc ctt ggc cag gca ttg gaa gtt gtc att cag        4359
Asn Asn Lys Pro Val Ser Leu Gly Gln Ala Leu Glu Val Val Ile Gln
```

```
                415                 420                 425

tta caa gag aag cat gtc aaa gat gag cag att gaa cat tgg aag aag      4407
Leu Gln Glu Lys His Val Lys Asp Glu Gln Ile Glu His Trp Lys Lys
        430                 435                 440

ata gtg aaa act cag gaa gaa ttg aaa gaa ctt ctt aat aag atg gta      4455
Ile Val Lys Thr Gln Glu Glu Leu Lys Glu Leu Leu Asn Lys Met Val
    445                 450                 455

aat ttg aaa gag aaa att aaa gaa ctc cat cag caa tac aaa gaa gca      4503
Asn Leu Lys Glu Lys Ile Lys Glu Leu His Gln Gln Tyr Lys Glu Ala
460                 465                 470                 475

tct gaa gta aag cca ccc aga gat att act gcc gag ttc tta gtg aaa      4551
Ser Glu Val Lys Pro Pro Arg Asp Ile Thr Ala Glu Phe Leu Val Lys
                480                 485                 490

agc aaa cac agg gat ctg acc gcc cta tgc aag gaa tat gat gaa tta      4599
Ser Lys His Arg Asp Leu Thr Ala Leu Cys Lys Glu Tyr Asp Glu Leu
            495                 500                 505

gct gaa aca caa gga aag cta gaa gaa aaa ctt cag gag ttg gaa gcg      4647
Ala Glu Thr Gln Gly Lys Leu Glu Glu Lys Leu Gln Glu Leu Glu Ala
        510                 515                 520

aat ccc cca agt gat gta tat ctc tca tca aga gac aga caa ata ctt      4695
Asn Pro Pro Ser Asp Val Tyr Leu Ser Ser Arg Asp Arg Gln Ile Leu
    525                 530                 535

gat tgg cat ttt gca aat ctt gaa ttt gct aat gcc aca cct ctc tca      4743
Asp Trp His Phe Ala Asn Leu Glu Phe Ala Asn Ala Thr Pro Leu Ser
540                 545                 550                 555

act ctc tcc ctt aag cac tgg gat cag gat gat gac ttt gag ttc act      4791
Thr Leu Ser Leu Lys His Trp Asp Gln Asp Asp Asp Phe Glu Phe Thr
                560                 565                 570

ggc agc cac ctg aca gta agg aat ggc tac tcg tgt gtg cct gtg gct      4839
Gly Ser His Leu Thr Val Arg Asn Gly Tyr Ser Cys Val Pro Val Ala
            575                 580                 585

tta gca gaa ggc cta gac att aaa ctg aat aca gca gtg cga cag gtt      4887
Leu Ala Glu Gly Leu Asp Ile Lys Leu Asn Thr Ala Val Arg Gln Val
        590                 595                 600

cgc tac acg gct tca gga tgt gaa gtg ata gct gtg aat acc cgc tcc      4935
Arg Tyr Thr Ala Ser Gly Cys Glu Val Ile Ala Val Asn Thr Arg Ser
    605                 610                 615

acg agt caa acc ttt att tat aaa tgc gac gca gtt ctc tgt acc ctt      4983
Thr Ser Gln Thr Phe Ile Tyr Lys Cys Asp Ala Val Leu Cys Thr Leu
620                 625                 630                 635

ccc ctg ggt gtg ctg aag cag cag cca cca gcc gtt cag ttt gtg cca      5031
Pro Leu Gly Val Leu Lys Gln Gln Pro Pro Ala Val Gln Phe Val Pro
                640                 645                 650

cct ctc cct gag tgg aaa aca tct gca gtc caa agg atg gga ttt ggc      5079
Pro Leu Pro Glu Trp Lys Thr Ser Ala Val Gln Arg Met Gly Phe Gly
            655                 660                 665

aac ctt aac aag gtg gtg ttg tgt ttt gat cgg gtg ttc tgg gat cca      5127
Asn Leu Asn Lys Val Val Leu Cys Phe Asp Arg Val Phe Trp Asp Pro
        670                 675                 680

agt gtc aat ttg ttc ggg cat gtt ggc agt acg act gcc agc agg ggt      5175
Ser Val Asn Leu Phe Gly His Val Gly Ser Thr Thr Ala Ser Arg Gly
    685                 690                 695
```

```
gag ctc ttc ctc ttc tgg aac ctc tat aaa gct cca ata ctg ttg gca        5223
Glu Leu Phe Leu Phe Trp Asn Leu Tyr Lys Ala Pro Ile Leu Leu Ala
700                 705                 710                 715

cta gtg gca gga gaa gct gct ggt atc atg gaa aac ata agt gac gat        5271
Leu Val Ala Gly Glu Ala Ala Gly Ile Met Glu Asn Ile Ser Asp Asp
                720                 725                 730

gtg att gtt ggc cga tgc ctg gcc att ctc aaa ggg att ttt ggt agc        5319
Val Ile Val Gly Arg Cys Leu Ala Ile Leu Lys Gly Ile Phe Gly Ser
            735                 740                 745

agt gca gta cct cag ccc aaa gaa act gtg gtg tct cgt tgg cgt gct        5367
Ser Ala Val Pro Gln Pro Lys Glu Thr Val Val Ser Arg Trp Arg Ala
        750                 755                 760

gat ccc tgg gct cgg ggc tct tat tcc tat gtt gct gca gga tca tct        5415
Asp Pro Trp Ala Arg Gly Ser Tyr Ser Tyr Val Ala Ala Gly Ser Ser
    765                 770                 775

gga aat gac tat gat tta atg gct cag cca atc act cct ggc ccc tcg        5463
Gly Asn Asp Tyr Asp Leu Met Ala Gln Pro Ile Thr Pro Gly Pro Ser
780                 785                 790                 795

att cca ggt gcc cca cag ccg att cca cga ctc ttc ttt gcg gga gaa        5511
Ile Pro Gly Ala Pro Gln Pro Ile Pro Arg Leu Phe Phe Ala Gly Glu
                800                 805                 810

cat acg atc cgt aac tac cca gcc aca gtg cat ggt gct ctg ctg agt        5559
His Thr Ile Arg Asn Tyr Pro Ala Thr Val His Gly Ala Leu Leu Ser
            815                 820                 825

ggg ctg cga gaa gcg gga aga att gca gac cag ttt ttg ggg gcc atg        5607
Gly Leu Arg Glu Ala Gly Arg Ile Ala Asp Gln Phe Leu Gly Ala Met
        830                 835                 840

tat acg ctg cct cgc cag gcc aca cca ggt gtt cct gca cag cag tcc        5655
Tyr Thr Leu Pro Arg Gln Ala Thr Pro Gly Val Pro Ala Gln Gln Ser
    845                 850                 855

cca agc atg tga gctaggatct tattaaagca gaacttgttt attgcagctt           5707
Pro Ser Met
860

ataatggtta caaataaagc aatagcatca caaatttcac aaataaagca tttttttcac     5767

tgcattctag ttgtggtttg tccaaactca tcaatgtatc ttatcatgtc tggtcgactc     5827

tagcagtgaa agtctgcaat gaattcgagt tggcgcgcct gtcattctaa atctctcttt     5887

cagcctaaag ctttttcccc gtatcccccc aggtgtctgc aggctcaaag agcagcgaga     5947

agcgttcaga ggaaagcgat cccgtgccac cttccccgtg cccgggctgt ccccgcacgc     6007

tgccggctcg gggatgcggg gggagcgccg gaccggagcg gagccccggg cggctcgctg     6067

ctgcccccta gcgggggagg gacgtaatta catccctggt gggctttggg aggggggctg     6127

tccccgtgag ctcccaggcg cgcctgtcat tctaaatctc tctttcagcc taaagctttt     6187

tccccgtatc cccccaggtg tctgcaggct caaagagcag cgagaagcgt tcagaggaaa     6247

gcgatcccgt gccaccttcc ccgtgcccgg gctgtccccg cacgctgccg gctcggggat     6307

gcggggggag cgccggaccg gagcggagcc ccgggcggct cgctgctgcc ccctagcggg     6367

ggagggacgt aattacatcc ctgggggctt tggggggggg ctgtccccgt gagctcccag     6427
```

gcgcgccaac tcgctagagg taccggttgt taacgttagc cggctacgta tactccggaa 6487
tattaatagg cctaggatgc atatggcggc cgccaccgcg gtggagctcc agcttttgtt 6547
gcgcgcttgg cgtaatcatg gtcatagctg tttcctgtgt gaaattgtta tccgctcaca 6607
attccacaca acatacgagc cggaagcata aagtgtaaag cctggggtgc ctaatgagtg 6667
agctaactca cattaattgc gttgcgctca ctgcccgctt tccagtcggg aaacctgtcg 6727
tgccagctgc attaatgaat cggccaacgc gcggggagag gcggtttgcg tattgggcgc 6787
tcttccgctt cctcgctcac tgactcgctg cgctcggtcg ttcggctgcg gcgagcggta 6847
tcagctcact caaaggcggt aatacggtta tccacagaat caggggataa cgcaggaaag 6907
aacatgtgag caaaaggcca gcaaaaggcc aggaaccgta aaaaggccgc gttgctggcg 6967
tttttccata ggctccgccc ccctgacgag catcacaaaa atcgacgctc aagtcagagg 7027
tggcgaaacc cgacaggact ataaagatac caggcgtttc cccctggaag ctccctcgtg 7087
cgctctcctg ttccgaccct gccgcttacc ggatacctgt ccgcctttct cccttcggga 7147
agcgtggcgc tttctcatag ctcacgctgt aggtatctca gttcggtgta ggtcgttcgc 7207
tccaagctgg gctgtgtgca cgaacccccc gttcagcccg accgctgcgc cttatccggt 7267
aactatcgtc ttgagtccaa cccggtaaga cacgacttat cgccactggc agcagccact 7327
ggtaacagga ttagcagagc gaggtatgta ggcggtgcta cagagttctt gaagtggtgg 7387
cctaactacg gctacactag aaggacagta tttggtatct gcgctctgct gaagccagtt 7447
accttcggaa aaagagttgg tagctcttga tccggcaaac aaaccaccgc tggtagcggt 7507
ggtttttttg tttgcaagca gcagattacg cgcagaaaaa aaggatctca agaagatcct 7567
ttgatctttt ctacggggtc tgacgctcag tggaacgaaa actcacgtta agggattttg 7627
gtcatgagat tatcaaaaag gatcttcacc tagatccttt taaattaaaa atgaagtttt 7687
aaatcaatct aaagtatata tgagtaaact tggtctgaca gttaccaatg cttaatcagt 7747
gaggcaccta tctcagcgat ctgtctattt cgttcatcca tagttgcctg actccccgtc 7807
gtgtagataa ctacgatacg ggagggctta ccatctggcc ccagtgctgc aatgataccg 7867
cgagacccac gctcaccggc tccagattta tcagcaataa accagccagc cggaagggcc 7927
gagcgcagaa gtggtcctgc aactttatcc gcctccatcc agtctattaa ttgttgccgg 7987
gaagctagag taagtagttc gccagttaat agtttgcgca acgttgttgc cattgctaca 8047
ggcatcgtgg tgtcacgctc gtcgtttggt atggcttcat tcagctccgg ttcccaacga 8107
tcaaggcgag ttacatgatc ccccatgttg tgcaaaaaag cggttagctc cttcggtcct 8167
ccgatcgttg tcagaagtaa gttggccgca gtgttatcac tcatggttat ggcagcactg 8227
cataattctc ttactgtcat gccatccgta agatgctttt ctgtgactgg tgagtactca 8287
accaagtcat tctgagaata gtgtatgcgg cgaccgagtt gctcttgccc ggcgtcaata 8347
cgggataata ccgcgccaca tagcagaact ttaaaagtgc tcatcattgg aaaacgttct 8407
tcggggcgaa aactctcaag gatcttaccg ctgttgagat ccagttcgat gtaacccact 8467
cgtgcaccca actgatcttc agcatctttt actttcacca gcgtttctgg gtgagcaaaa 8527

acaggaaggc aaaatgccgc aaaaaaggga ataagggcga cacggaaatg ttgaatactc 8587

atactcttcc tttttcaata ttattgaagc atttatcagg gttattgtct catgagcgga 8647

tacatatttg aatgtattta gaaaaataaa caaatagggg ttccgcgcac atttccccga 8707

aaagtgccac 8717

<210> 7
<211> 862
<212> PRT
<213> artificial sequence

<220>
<223> Synthetic Construct

<400> 7

```
Met Asp Tyr Lys Asp Asp Asp Asp Lys Glu Phe Leu Ser Gly Lys Lys
1               5                   10                  15

Ala Ala Ala Ala Ala Ala Ala Ala Ala Ala Ala Ala Thr Gly Thr Glu
            20                  25                  30

Ala Gly Pro Gly Thr Ala Gly Gly Ser Glu Asn Gly Ser Glu Val Ala
        35                  40                  45

Ala Gln Pro Ala Gly Leu Ser Gly Pro Ala Glu Val Gly Pro Gly Ala
    50                  55                  60

Val Gly Glu Arg Thr Pro Arg Lys Lys Glu Pro Pro Arg Ala Ser Pro
65                  70                  75                  80

Pro Gly Gly Leu Ala Glu Pro Pro Gly Ser Ala Gly Pro Gln Ala Gly
                85                  90                  95

Pro Thr Val Val Pro Gly Ser Ala Thr Pro Met Glu Thr Gly Ile Ala
            100                 105                 110

Glu Thr Pro Glu Gly Arg Arg Thr Ser Arg Arg Lys Arg Ala Lys Val
        115                 120                 125

Glu Tyr Arg Glu Met Asp Glu Ser Leu Ala Asn Leu Ser Glu Asp Glu
    130                 135                 140

Tyr Tyr Ser Glu Glu Glu Arg Asn Ala Lys Ala Glu Lys Glu Lys Lys
145                 150                 155                 160

Leu Pro Pro Pro Pro Pro Gln Ala Pro Pro Glu Glu Glu Asn Glu Ser
                165                 170                 175

Glu Pro Glu Glu Pro Ser Gly Val Glu Gly Ala Ala Phe Gln Ser Arg
            180                 185                 190

Leu Pro His Asp Arg Met Thr Ser Gln Glu Ala Ala Cys Phe Pro Asp
```

```
        195                 200                 205

Ile Ile Ser Gly Pro Gln Gln Thr Gln Lys Val Phe Leu Phe Ile Arg
    210                 215                 220

Asn Arg Thr Leu Gln Leu Trp Leu Asp Asn Pro Lys Ile Gln Leu Thr
225                 230                 235                 240

Phe Glu Ala Thr Leu Gln Gln Leu Glu Ala Pro Tyr Asn Ser Asp Thr
                245                 250                 255

Val Leu Val His Arg Val His Ser Tyr Leu Glu Arg His Gly Leu Ile
            260                 265                 270

Asn Phe Gly Ile Tyr Lys Arg Ile Lys Pro Leu Pro Thr Lys Lys Thr
        275                 280                 285

Gly Lys Val Ile Ile Ile Gly Ser Gly Val Ser Gly Leu Ala Ala Ala
    290                 295                 300

Arg Gln Leu Gln Ser Phe Gly Met Asp Val Thr Leu Leu Glu Ala Arg
305                 310                 315                 320

Asp Arg Val Gly Gly Arg Val Ala Thr Phe Arg Lys Gly Asn Tyr Val
                325                 330                 335

Ala Asp Leu Gly Ala Met Val Val Thr Gly Leu Gly Gly Asn Pro Met
            340                 345                 350

Ala Val Val Ser Lys Gln Val Asn Met Glu Leu Ala Lys Ile Lys Gln
        355                 360                 365

Lys Cys Pro Leu Tyr Glu Ala Asn Gly Gln Ala Val Pro Lys Glu Lys
    370                 375                 380

Asp Glu Met Val Glu Gln Glu Phe Asn Arg Leu Leu Glu Ala Thr Ser
385                 390                 395                 400

Tyr Leu Ser His Gln Leu Asp Phe Asn Val Leu Asn Asn Lys Pro Val
                405                 410                 415

Ser Leu Gly Gln Ala Leu Glu Val Val Ile Gln Leu Gln Glu Lys His
            420                 425                 430

Val Lys Asp Glu Gln Ile Glu His Trp Lys Lys Ile Val Lys Thr Gln
        435                 440                 445

Glu Glu Leu Lys Glu Leu Leu Asn Lys Met Val Asn Leu Lys Glu Lys
    450                 455                 460

Ile Lys Glu Leu His Gln Gln Tyr Lys Glu Ala Ser Glu Val Lys Pro
465                 470                 475                 480
```

```
Pro Arg Asp Ile Thr Ala Glu Phe Leu Val Lys Ser Lys His Arg Asp
                485                 490                 495

Leu Thr Ala Leu Cys Lys Glu Tyr Asp Glu Leu Ala Glu Thr Gln Gly
            500                 505                 510

Lys Leu Glu Glu Lys Leu Gln Glu Leu Glu Ala Asn Pro Pro Ser Asp
        515                 520                 525

Val Tyr Leu Ser Ser Arg Asp Arg Gln Ile Leu Asp Trp His Phe Ala
    530                 535                 540

Asn Leu Glu Phe Ala Asn Ala Thr Pro Leu Ser Thr Leu Ser Leu Lys
545                 550                 555                 560

His Trp Asp Gln Asp Asp Asp Phe Glu Phe Thr Gly Ser His Leu Thr
                565                 570                 575

Val Arg Asn Gly Tyr Ser Cys Val Pro Val Ala Leu Ala Glu Gly Leu
            580                 585                 590

Asp Ile Lys Leu Asn Thr Ala Val Arg Gln Val Arg Tyr Thr Ala Ser
        595                 600                 605

Gly Cys Glu Val Ile Ala Val Asn Thr Arg Ser Thr Ser Gln Thr Phe
    610                 615                 620

Ile Tyr Lys Cys Asp Ala Val Leu Cys Thr Leu Pro Leu Gly Val Leu
625                 630                 635                 640

Lys Gln Gln Pro Pro Ala Val Gln Phe Val Pro Pro Leu Pro Glu Trp
                645                 650                 655

Lys Thr Ser Ala Val Gln Arg Met Gly Phe Gly Asn Leu Asn Lys Val
            660                 665                 670

Val Leu Cys Phe Asp Arg Val Phe Trp Asp Pro Ser Val Asn Leu Phe
        675                 680                 685

Gly His Val Gly Ser Thr Thr Ala Ser Arg Gly Glu Leu Phe Leu Phe
    690                 695                 700

Trp Asn Leu Tyr Lys Ala Pro Ile Leu Leu Ala Leu Val Ala Gly Glu
705                 710                 715                 720

Ala Ala Gly Ile Met Glu Asn Ile Ser Asp Asp Val Ile Val Gly Arg
                725                 730                 735

Cys Leu Ala Ile Leu Lys Gly Ile Phe Gly Ser Ser Ala Val Pro Gln
            740                 745                 750
```

Pro Lys Glu Thr Val Val Ser Arg Trp Arg Ala Asp Pro Trp Ala Arg
755 760 765

Gly Ser Tyr Ser Tyr Val Ala Ala Gly Ser Ser Gly Asn Asp Tyr Asp
770 775 780

Leu Met Ala Gln Pro Ile Thr Pro Gly Pro Ser Ile Pro Gly Ala Pro
785 790 795 800

Gln Pro Ile Pro Arg Leu Phe Phe Ala Gly Glu His Thr Ile Arg Asn
805 810 815

Tyr Pro Ala Thr Val His Gly Ala Leu Leu Ser Gly Leu Arg Glu Ala
820 825 830

Gly Arg Ile Ala Asp Gln Phe Leu Gly Ala Met Tyr Thr Leu Pro Arg
835 840 845

Gln Ala Thr Pro Gly Val Pro Ala Gln Gln Ser Pro Ser Met
850 855 860

<210> 8
<211> 20
<212> DNA
<213> artificial sequence

<220>
<223> primer 1

<400> 8
aatgccttcg aattcagcac 20

<210> 9
<211> 20
<212> DNA
<213> artificial sequence

<220>
<223> primer 2

<400> 9
ccttgtcatc gtcgtccttg 20

<210> 10
<211> 18
<212> DNA
<213> artificial sequence

<220>
<223> primer 3

<400> 10
gactacaagg acgacgat 18

<210> 11
<211> 31
<212> DNA
<213> artificial sequence

```
<220>
<223>  primer 4

<400>  11
ccgctcgagt cagctttcat ccatctctct g                                    31
```